(19) Europäisches Patentamt
European Patent Office
Office européen des brevets

(11) EP 4 606 801 A1

(12) EUROPEAN PATENT APPLICATION
published in accordance with Art. 153(4) EPC

(43) Date of publication:
**27.08.2025 Bulletin 2025/35**

(21) Application number: **23879187.5**

(22) Date of filing: **19.10.2023**

(51) International Patent Classification (IPC):
***C07D 471/04*** (2006.01) ***C07D 403/14*** (2006.01)
***C07D 401/14*** (2006.01) ***A61K 31/444*** (2006.01)
***A61P 35/00*** (2006.01) ***A61P 35/02*** (2006.01)
***A61P 35/04*** (2006.01)

(52) Cooperative Patent Classification (CPC):
**A61K 31/444; A61K 31/498; A61P 35/00; A61P 35/02; A61P 35/04; C07D 401/14; C07D 403/14; C07D 471/04**

(86) International application number:
**PCT/CN2023/125496**

(87) International publication number:
**WO 2024/083201 (25.04.2024 Gazette 2024/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.10.2022 CN 202211291843**
**16.08.2023 CN 202311030970**

(71) Applicant: **Chengdu Zenitar Biomedical Technology Co., Ltd.**
**Chengdu, Sichuan 610000 (CN)**

(72) Inventors:
- **WANG, Taijin**
**Chengdu, Sichuan 610000 (CN)**
- **JIA, Tao**
**Chengdu, Sichuan 610000 (CN)**
- **BAI, Zhongyou**
**Chengdu, Sichuan 610000 (CN)**
- **LI, Gang**
**Chengdu, Sichuan 610000 (CN)**

(74) Representative: **Gulde & Partner**
**Patent- und Rechtsanwaltskanzlei mbB**
**Berliner Freiheit 2**
**10785 Berlin (DE)**

# (54) FUSED HETEROCYCLIC COMPOUND AND USE THEREOF

(57) The present invention discloses a series of fused heterocyclic compounds and uses thereof, belonging to the technical field of chemical medicine. The fused heterocyclic compound represented by Formula II and provided by the present invention can serve as a PARP1 inhibitor, exhibiting advantages of high activity and high selectivity, as well as excellent pharmacokinetic properties and outstanding safety.

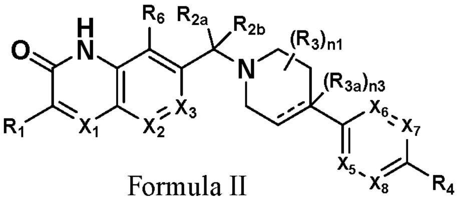

Formula II

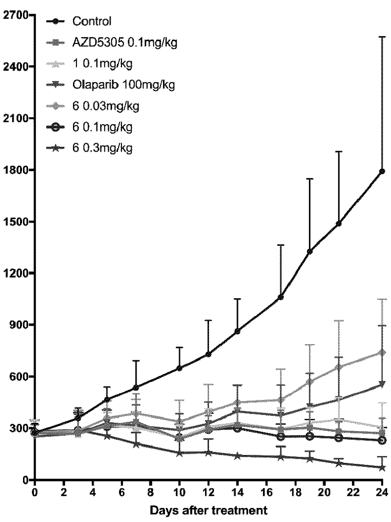


Fig. 1

EP 4 606 801 A1

## Description

### Technical Field

The present invention belongs to the field of chemical medicine and relates to a series of fused heterocyclic compounds and uses thereof.

### Background

During the growth process of a cell, its DNA is constantly damaged by various internal and external adverse factors. Among DNA damages, the most severe types are single-strand breaks (SSBs) and double-strand breaks (DSBs), with SSBs being more prevalent. If these breaks are not repaired timely and accurately, they can lead to genomic instability, which may subsequently cause carcinogenesis or even directly result in cell death. For single-strand breaks in DNA, its repair primarily relies on PARP enzymes. For double-strand breaks, there are two main repair pathways for double-stranded DNA: non-homologous end joining (NHEJ) repair and homologous recombination (HR) repair. Homologous recombination repair is a high-fidelity, error-free repair mechanism and the primary pathway for double-stranded DNA repair. Homologous recombination repair involves numerous proteins, among which the most well-known are BRCA proteins. Two studies in 2005 (Farmer H, McCabe N, et al. Targeting the DNA repair defect in BRCA mutant cells as a therapeutic strategy[J]. Nature, 2005, 434(7035):917-921. Bryant, H., Schultz, N., Thomas, H. et al. Specific killing of BRCA2-deficient tumors with inhibitors of poly(ADP-ribose) polymerase. Nature 434, 913-917 (2005)) demonstrated that tumor cells lacking BRCA1 or BRCA2 are selectively inhibited by PARP inhibitors. Based on this research result, scholars proposed the concept of synthetic lethality: The loss of either of the two genes, BRCA or PARP, is not fatal by itself, but the simultaneous inactivation of both leads to cell death. Based on the synthetic lethality theory, PARP inhibitors (PARPi) have been developed for selective targeting of BRCA1/2-mutated cancer cells.

PARP inhibitors have demonstrated excellent clinical efficacy in patients with homologous recombination-deficient cancers. However, whether administered as monotherapy or in combination therapies, hematologic toxicity (anemia, neutropenia, and thrombocytopenia) and other toxicities limit the application of these drugs. Relevant studies have shown (Harris P A, Boloor A, Cheung M, et al. Discovery of 5-[[4-[(2,3-dimethyl-2H-indazol-6-yl)methylamino]-2-pyrimidinyl]amino]-2-methyl-benzenesulfonamide (Pazopanib), a novel and potent vascular endothelial growth factor receptor inhibitor. [J]. Journal of Medicinal Chemistry, 2008, 51(15):4632) that these adverse reactions may originate from the inhibition of PARP2 by marketed PARP inhibitors, despite PARP2 not being essential for therapeutic efficacy. Highly selective PARP1 inhibitors can reduce hematological toxicity, enhance the therapeutic safety window, and increase the potential for combination use with other chemotherapy drugs or targeted therapies.

Therefore, there remains an unmet clinical need for effective and safe PARP inhibitors, particularly those selective for PARP1. The novel PARP1 inhibitors described in the present invention unexpectedly exhibit significantly higher selectivity for PARP1 compared to other PARP family members (e.g. PARP2, PARP3, PARP5a, and PARP6), and can be used for treating PARP function-related diseases.

### Summary

An object of the present invention is to provide a class of fused heterocyclic compounds and uses thereof, for achieving highly selective and effective prevention or treatment of PARP function-associated diseases.

In a first aspect, the present invention provides a compound represented by Formula II or a pharmaceutically acceptable form thereof, wherein Formula II is as follows:

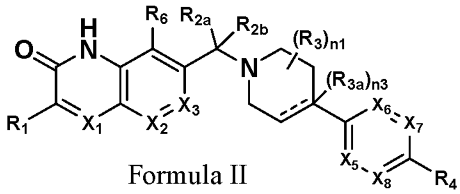

Formula II

Where,

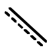 represents a single bond or a double bond;

$R_1$ is selected from $C_{1-4}$ deuterated alkyl;

$X_1$ is selected from N or $C(R_{5a})$, $X_2$ is selected from N or $C(R_{5b})$, and $X_3$ is selected from N or $C(R_{5c})$, with exactly one of

$X_1$, $X_2$, and $X_3$ selected from N;

$R_{2a}$ and $R_{2b}$ are independently selected from hydrogen, deuterium, methyl, or deuteromethyl;

$R_3$ is selected from at least one of deuterium, fluorine, $C_{1-4}$ alkyl, or $C_{1-4}$ deuterated alkyl;

$R_{3a}$ is selected from hydrogen, deuterium, fluorine, hydroxyl, cyano, $C_{1-4}$ alkyl, $C_{1-4}$ fluoroalkyl, or $C_{1-4}$ alkoxy;

$R_4$ is selected from hydrogen, halogen, cyano, $C_{1-4}$ alkyl, $C_{1-4}$ deuterated alkyl, $C_{1-4}$ fluoroalkyl, or -CONHR$_7$;

$R_{5a}$, $R_{5a}$ and $R_{5c}$ are independently selected from hydrogen, fluorine, chlorine, $C_{1-4}$ alkyl, $C_{1-4}$ deuterated alkyl, $C_{1-4}$ fluoroalkyl, $C_{1-4}$ alkoxy or $C_{1-4}$ fluoroalkoxy;

$R_6$ is selected from hydrogen, fluorine, chlorine, $C_{1-4}$ alkyl, $C_{1-4}$ fluoroalkyl, or $C_{1-4}$ deuterated alkyl;

$R_7$ is selected from hydrogen, $C_{1-4}$ alkyl, $C_{1-4}$ deuterated alkyl, $C_{1-4}$ fluoroalkyl, or a 3-6 membered cycloalkyl;

$X_5$ is selected from nitrogen or C($R_{9a}$), $X_6$ is selected from nitrogen or C($R_{9b}$), $X_7$ is selected from nitrogen or C($R_{9c}$), and $X_8$ is selected from nitrogen or C($R_{9d}$);

$R_{9a}$, $R_{9b}$, $R_{9c}$, and $R_{9d}$ are independently selected from hydrogen, fluorine, chlorine, cyano, $C_{1-4}$ alkyl, $C_{1-4}$ deuterated alkyl, $C_{1-4}$ fluoroalkyl, $C_{1-4}$ alkoxy, or $C_{1-4}$ fluoroalkoxy;

n1 is an integer selected from 0 to 8;

n3 is independently selected from 0 or 1;

The pharmaceutically acceptable form is selected from the group consisting of a pharmaceutically acceptable salt, ester, stereoisomer, polymorph, solvate, N-oxide, isotopically labeled compound, metabolite, or prodrug.

In some embodiments of the invention, for the compound represented by Formula II above or a pharmaceutically acceptable form thereof, $R_1$ is selected from deuteromethyl or deuterated ethyl.

In some preferred embodiments of the present invention, for the compound represented by Formula II or a pharmaceutically acceptable form thereof described above, $R_1$ is selected from -$CD_2CD_3$ or -$CH_2CD_3$.

In some embodiments of the invention, for the compound represented by Formula II above or a pharmaceutically acceptable form thereof, $R_3$ is selected from at least one of deuterium, methyl, deuteromethyl, fluoromethyl, or methoxy.

In some preferred embodiments of the present invention, $R_3$ in the compound represented by Formula II above or a pharmaceutically acceptable form thereof is selected from deuterium or methyl.

In some embodiments of the present invention, for the compound or a pharmaceutically acceptable form thereof represented by Formula II above, when 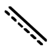 is a double bond, $R_{3a}$ is absent; when 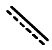 is a single bond, $R_{3a}$ is selected from hydrogen and deuterium.

In some embodiments of the invention, for the compound represented by Formula II or a pharmaceutically acceptable form thereof described above, $R_4$ is selected from fluorine, chlorine, cyano, $C_{1-4}$ alkyl, $C_{1-4}$ deuterated alkyl, $C_{1-4}$ fluoroalkyl, $C_{1-4}$ alkylaminocarbonyl, $C_{1-4}$ deuterated alkylaminocarbonyl, $C_{1-4}$ fluoroalkylaminocarbonyl, or 3-6 membered cycloalkylaminocarbonyl.

In some preferred embodiments of the invention, for the compound represented by Formula II or a pharmaceutically acceptable form thereof described above, $R_4$ is selected from fluoro, cyano, methylaminocarbonyl, deuteriomethylaminocarbonyl, and cyclopropylaminocarbonyl.

In some preferred embodiments of the invention, for the compound or a pharmaceutically acceptable form thereof represented by Formula II above, $R_6$ is selected from hydrogen, fluorine, chlorine, or methyl.

In some embodiments of the invention, for the compound represented by Formula II above or a pharmaceutically acceptable form thereof, $R_{5a}$, $R_{5b}$, and $R_{5c}$ are independently selected from hydrogen, fluorine, chlorine, or methyl.

In some embodiments of the invention, in addition to the compound represented by Formula II above or a pharmaceutically acceptable form thereof, the invention also provides the compound having the structure represented by Formula VI-1:

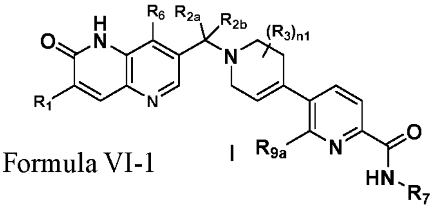

Formula VI-1

Where,

$R_6$ is selected from hydrogen or fluorine;

$R_{9a}$ is selected from hydrogen, fluorine, chlorine, cyano, $C_{1-4}$ alkyl, $C_{1-4}$ deuterated alkyl, or $C_{1-4}$ fluoroalkyl;

n1 is an integer selected from 0 to 6.

In some embodiments of the invention, in addition to the compound represented by Formula II above or a pharmaceutically acceptable form thereof, the invention also provides a compound having the structure represented by Formula VI-3:

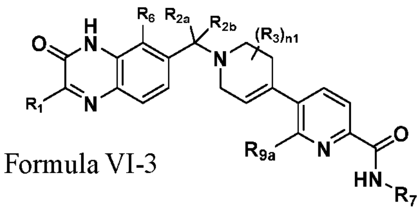

Formula VI-3

Where,

- $R_6$ is selected from hydrogen or fluorine;
- $R_{9a}$ is selected from hydrogen, fluorine, chlorine, cyano, $C_{1-4}$ alkyl, $C_{1-4}$ deuterated alkyl, or $C_{1-4}$ fluoroalkyl;
- n1 is an integer selected from 0 to 6.

In some more preferred embodiments of the present invention, there are provided the following compounds, pharmaceutically acceptable salts, esters, stereoisomers, polymorphs, solvates, N-oxides, isotopically labeled compounds, metabolites, or prodrugs thereof:

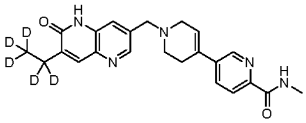 , 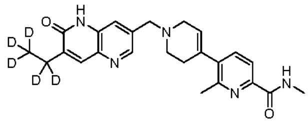 , 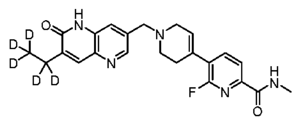 ,

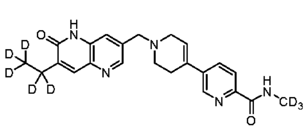 , 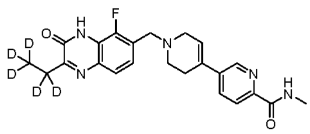 or 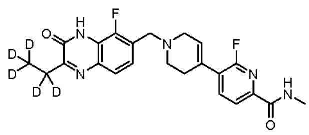 .

In some more preferred embodiments of the present invention, there are provided the following compounds, pharmaceutically acceptable salts, esters, stereoisomers, polymorphs, solvates, N-oxides, isotopically labeled compounds, metabolites, or prodrugs thereof:

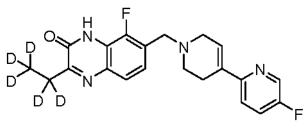 or 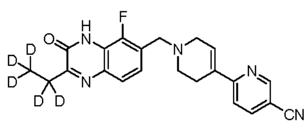 .

In some more preferred embodiments of the present invention, there are provided the following compounds, pharmaceutically acceptable salts, esters, stereoisomers, polymorphs, solvates, N-oxides, isotopically labeled compounds, metabolites, or prodrugs thereof:

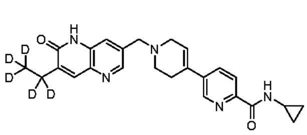 , 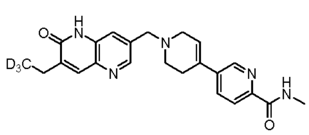 , 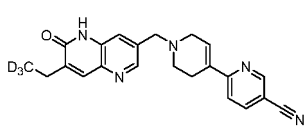

or

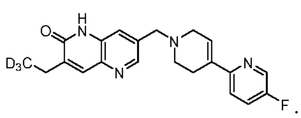 .

In a second aspect, the present invention provides a pharmaceutical composition comprising: the aforementioned compound (represented by Formula II, FormulaVI-1, or FormulaVI-3) or a pharmaceutically acceptable salt, ester, stereoisomer, tautomer, polymorph, solvate, N-oxide, isotopically labeled compound, metabolite, or prodrug thereof, as an active ingredient; and a pharmaceutically acceptable carrier.

A further object of the present invention is to provide a method for preparing the pharmaceutical composition of the present invention, comprising combining: any compound of Formula II, FormulaVI-1, or FormulaVI-3, or a pharmaceutically acceptable form thereof, or a mixture thereof; and one or more pharmaceutically acceptable carriers.

The pharmaceutically acceptable carrier that can be used in the pharmaceutical compositions of the present invention is a pharmaceutically acceptable carrier, examples of suitable pharmaceutically acceptable carriers are as described in Remington's Pharmaceutical Sciences (2005).

The pharmaceutical composition can be administered in any form as long as it achieves the prevention, alleviation, inhibition, or cure of symptoms in human or animal patients. For example, various suitable dosage forms can be prepared according to the route of administration.

In other embodiments, the administration of the compound or pharmaceutical composition of the present invention can be combined with another therapeutic method. The additional therapeutic methods can be selected from, but are not limited to: radiotherapy, chemotherapy, immunotherapy, or combinations thereof.

The present invention further relates to a pharmaceutical formulation comprising any compound represented by Formula II, FormulaVI-1, or FormulaVI-3, or a pharmaceutically acceptable form thereof, or a mixture thereof as an active ingredient, or the pharmaceutical composition of the present invention. In some embodiments, the formulation is in the form of a solid formulation, a semi-solid formulation, a liquid formulation, or a gaseous formulation.

A further objective of the present invention is to provide a product, for example, in the form of a kit. The products referred to herein are intended to include, but are not limited to, kits and packaging. The product of the present invention comprises: (a) a first container; (b) a pharmaceutical composition located in the first container, wherein the composition comprises: a first therapeutic agent comprising any compound represented by Formula II, FormulaVI-1, or FormulaVI-3, or a pharmaceutically acceptable form thereof, or a mixture thereof; (c) optionally, a packaging specification indicating that the pharmaceutical composition can be used for treating a neoplastic disorder (as defined below); and (d) a second container.

The first container is a container for containing a pharmaceutical composition. This container can be used for preparation, storage, transportation, and/or individual/bulk sales. The first container is intended to encompass bottles, cans, vials, flasks, syringes, tubes (e.g., for cream products), or any other container for preparing, containing, storing, or dispensing pharmaceutical products.

The second container is a container for accommodating the first container and optionally the packaging specification. Examples of the second container include, but are not limited to, boxes (such as cardboard boxes or plastic boxes), crates, cartons, bags (such as paper bags or plastic bags), pouches, and burlap sacks. The packaging specification may be physically adhered to the exterior of the first container via a tie, glue, staples, or another adhesive means, or it may be placed inside the second container without the need for any physical means of adhesion to the first container. Alternatively, the packaging specification is located outside the second container. When located outside the second container, it is preferable that the packaging specification be physically adhered via a tie, glue, staples, or another adhesion means. Alternatively, it may abut against or contact the exterior of the second container without the need for physical adhesion.

The packaging specification includes trademarks, labels, markings, and the like, which list information related to the pharmaceutical composition located within the first container. The information listed is typically determined by the regulatory agency governing the region where the product is intended for sale (e.g., the U.S. Food and Drug Administration). Preferably, the packaging specification specifically lists the indications for which the pharmaceutical composition is approved. The packaging specification may be made of any material from which information contained therein or thereon may be read. Preferably, the packaging specification is a printable material (such as paper, plastic, cardboard, foil, adhesive paper, plastic, and the like) on which the required information may be formed (e.g., printed or applied).

In a third aspect, the present invention provides for the use of the aforementioned compounds, compounds of Formula II, FormulaVI-1, or FormulaVI-3, as well as related specific compounds or pharmaceutically acceptable forms thereof, or the pharmaceutical compositions of the present invention, in the manufacture of a medicament for preventing or treating a PARP1 enzyme-related disease.

The present invention provides a method for preventing or treating a PARP1 enzyme-related disease, comprising administering to an individual in need thereof a compound represented by Formula II, FormulaVI-1, or FormulaVI-3, or a pharmaceutically acceptable form thereof, or a pharmaceutical composition of the present invention.

The present invention provides a compound represented by Formula II, FormulaVI-1, or FormulaVI-3, or a pharmaceutically acceptable form thereof, or a pharmaceutical composition of the present invention, for preventing or treating a PARP1 enzyme-related disease.

The present invention provides methods for preventing or treating a PARP1 enzyme-related disease using a

compound represented by Formula II, FormulaVI-1, or FormulaVI-3, or a pharmaceutically acceptable form thereof, or a pharmaceutical composition of the present invention in combination with additional therapeutic methods, which include but are not limited to radiotherapy, chemotherapy, immunotherapy, or combinations thereof.

In some embodiments, the PARP1 enzyme-related disease is a disease sensitive to or responsive to PARP1 enzyme inhibition.

In some embodiments, the PARP1 enzyme-related disease is a neoplastic disorder.

In some preferred embodiments, the neoplastic disorder lacks an HR-dependent DNA DSB repair pathway.

In some preferred embodiments, the neoplastic disorder comprises one or more types of cancer cells that have a reduced or absent ability to repair DNA DSBs through HR compared to normal cells.

In some preferred embodiments, the cancer cells have a BRCA1 or BRCA2 deficient phenotype.

In some embodiments, the PARP1 enzyme-related disease is a neoplastic disorder, including but not limited to solid and hematological malignancies. In further embodiments, the neoplastic disorder includes, but is not limited to, breast cancer, colorectal cancer, colon cancer, lung cancer (including small cell lung cancer, non-small cell lung cancer, and bronchoalveolar carcinoma), and prostate cancer, as well as cholangiocarcinoma, bone cancer, bladder cancer, head and neck cancer, renal cancer, liver cancer, cancer of gastrointestinal tissues, esophageal cancer, ovarian cancer, pancreatic cancer, skin cancer, testicular cancer, thyroid cancer, uterine cancer, cervical cancer, and vulvar cancer, as well as leukemias (including chronic lymphocytic leukemia (CLL), acute lymphocytic leukemia (ALL), and chronic myeloid leukemia (CML), multiple myeloma, or lymphomas.

In some preferred embodiments, the PARP1 enzyme-related disease is a breast cancer, an ovarian cancer, a pancreatic cancer, a prostate cancer, a hematological cancer, a gastrointestinal cancer, or a lung cancer.

In further preferred embodiments, the compounds of the present invention can be used in combination with radiotherapy and chemotherapy or immunotherapy for the prevention or treatment of cancer.

The beneficial effects of the present invention are as follows:

The present invention provides a novel class of highly active and highly selective PARP1 inhibitors capable of achieving at least one of the following technical effects: (1) high inhibitory activity against PARP1 enzyme; (2) selective inhibition of PARP1 enzyme with high selectivity for other enzymes in the PARP family such as PARP2, PARP5a, and PARP5b; (3) strong inhibitory activity against homologous recombination-defective tumor cells and weak inhibitory effect on non-homologous recombination-defective cells; (4) excellent pharmacokinetic properties (such as good bioavailability, suitable half-life, and duration of action); (5) excellent safety (lower toxicity and/or fewer side effects, and a broader therapeutic window), among others.

Definition of terms:

Unless otherwise defined herein below, all technical and scientific terms used in this document are intended to have the meanings as commonly understood by those skilled in the art. The terms "including", "comprising", "having", "containing", or "relating to" and other variant forms thereof in this document are inclusive or open-ended and do not exclude other unenumerated elements or method steps. Those skilled in the art shall understand that the aforementioned terms such as "including" encompass the meaning of "consisting of".

In the present invention, "one", "a", "the", "at least one", and "one or more" can be used interchangeably. Therefore, for example, a composition comprising "a" pharmaceutically acceptable excipient may be interpreted to mean that the composition includes "one or more" pharmaceutically acceptable excipients.

For example, the expression "$C_{1-4}$" shall be understood to cover any sub-ranges therein and each point value, such as $C_{2-4}$, $C_{3-4}$, $C_{1-2}$, $C_{1-3}$, $C_{1-4}$, and the like, as well as $C_1$, $C_2$, $C_3$, $C_4$, and the like.

Unless otherwise specified, 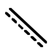 used herein represents a single bond or a double bond.

In the present invention, unless otherwise specified, halogen refers to fluorine, chlorine, bromine, or iodine.

In the present invention, unless otherwise specified, the term "alkyl" comprises straight-chain or branched monovalent saturated hydrocarbon groups. For example, alkyl groups include methyl, ethyl, propyl, isopropyl, n-butyl, isobutyl, sec-butyl, tert-butyl, n-pentyl, 3-(2-methyl) butyl, 2-pentyl, 2-methylbutyl, neopentyl, n-hexyl, 2-hexyl, 2-methylpentyl, and the like. Similarly, $C_{1-4}$ in "$C_{1-4}$ alkyl" refers to a group having 1, 2, 3, or 4 carbon atoms arranged in a straight or branched chain form.

In the present invention, unless otherwise specified, the term "cycloalkyl", "carbocyclic" or "cycloalkylene" refers to a saturated or partially saturated, monocyclic or polycyclic (such as bicyclic) non-aromatic hydrocarbon group. Common cycloalkyl groups include, but are not limited to, monocyclic cycloalkyl groups such as cyclopropyl, cyclobutyl, cyclopentyl, cyclohexyl, cycloheptyl, cyclooctyl, cyclononyl, cyclobutenyl, cyclopentenyl, cyclohexenyl, and the like; or bicyclic cycloalkyl groups, including fused, bridged, or spiro rings, such as bicyclo [1.1.1] pentyl, bicyclo [2.2.1] heptyl, bicyclo [3.2.1] octyl, bicyclo [5.2.0] nonyl, decahydronaphthyl, and the like. For example, the term "$C_{3-12}$ cycloalkyl" refers to a cycloalkyl group having 3 to 12 ring carbon atoms (such as 3, 4, 5, 6, 7, 8, 9, 10, 11, or 12). The cycloalkyl or cycloalkylene

group in the present invention is optionally substituted with one or more substituents described herein.

In the present invention, unless otherwise specified, the term "fluoroalkyl" refers to the aforementioned alkyl group, wherein one or more hydrogen atoms are substituted with fluorine atoms. For example, the term "$C_{1-4}$ fluoroalkyl" refers to a $C_{1-4}$ alkyl group optionally substituted with one or more (such as 1 to 3) fluorine atoms. Those skilled in the art shall understand that when there is more than one fluorine atom substituent, the fluorine atoms may be the same or different and may be located on the same or different C atoms. Examples of haloalkyl groups include, but are not limited to, $-CH_2F$, $-CHF_2$, $-CF_3$, $-C_2F_5$, $-CH_2CF_3$, $-CH_2CH_2CF_3$, and the like. The fluoroalkyl group in the present invention is optionally substituted with one or more substituents described herein.

The present invention also encompasses all pharmaceutically acceptable isotopically labeled compounds that are identical to the compounds of the present invention, except that one or more atoms are replaced by atoms having the same atomic number but an atomic mass or mass number different from the atomic mass or mass number predominant in nature. Examples of isotopes suitable for incorporation into the compounds of the present invention include, but are not limited to: isotopes of hydrogen (e.g., deuterium ($^{2}H$) and tritium ($^{3}H$)); isotopes of carbon (e.g., $^{13}C$ and $^{14}C$); isotopes of chlorine (e.g., $^{31}Cl$); isotopes of iodine (e.g., $^{125}I$); isotopes of nitrogen (e.g., $^{13}N$ and $^{15}N$); isotopes of oxygen (e.g., $^{17}O$ and $^{18}O$); isotopes of phosphorus (e.g., $^{32}P$); and isotopes of sulfur (e.g., $^{34}S$).

In present invention, the term "polymorph" refers to different solid crystalline phases of certain compounds of the present invention arising from the existence of two or more distinct molecular arrangements in the solid state. Certain compounds of the present invention may exist in more than one crystalline form, and the present invention is intended to encompass all such crystalline forms and mixtures thereof. Generally, crystallization results in the formation of solvates of the compounds of the present invention. As used in the present invention, the term "solvate" refers to an aggregate comprising one or more molecules of a compound of the present invention and one or more solvent molecules. The solvent may be water, in which case the solvate is a hydrate. Alternatively, the solvent may be an organic solvent. Therefore, the compounds of the present invention may exist as hydrates, including monohydrates, dihydrates, hemihydrates, sesquihydrates, trihydrates, tetrahydrates, and the like, as well as corresponding solvated forms. The compounds of the present invention may form true solvates, but in some cases, they may also retain merely non-stoichiometric water or a mixture of water combined with a portion of non-stoichiometric solvents. The compounds of the present invention may be reacted in a solvent or precipitated or crystallized from a solvent. Solvates of the compounds of the present invention are also encompassed within the scope of the present invention. The present invention also encompasses all possible crystalline forms or polymorphs of the compounds of the present invention, which may be a single polymorph or a mixture of more than one polymorph in any ratio.

In the present invention, the term "stereoisomers" refer to isomers formed resulting from the presence of at least one asymmetric center. In compounds possessing one or more (e.g., one, two, three, or four) asymmetric centers, they can give rise to racemic mixtures, single enantiomers, diastereomer mixtures, and individual diastereomers. Certain individual molecules may also exist as geometric isomers (cis/trans). Similarly, the compounds of the present invention may exist as a mixture of two or more structurally different forms in rapid equilibrium (commonly referred to as tautomers). Representative examples of tautomers include keto-enol tautomers, phenol-keto tautomers, nitroso-oxime tautomers, and imine-enamine tautomers. It is to be understood that the scope of the present invention encompasses all such isomers or mixtures thereof in any ratio (e.g., 60%, 65%, 70%, 75%, 80%, 85%, 90%, 95%, 96%, 97%, 98%, and 99%).

In the present invention, pharmaceutically acceptable salts encompass acid addition salts and base addition salts thereof. The suitable acid addition salt is formed from an acid that forms a pharmaceutically acceptable salt. The suitable base addition salts are formed from a base that forms a pharmaceutically acceptable salt. For an overview of suitable salts, reference may be made to, for example, Remington's Pharmaceutical Sciences, Mack Publishing Company, Easton, Pa., (2005); and Handbook of Pharmaceutical Salts: Properties, Selection, and Use, Stahl and Wermuth (Wiley-VCH, Weinheim, Germany, 2002). Methods for preparing pharmaceutically acceptable salts of the compounds of the present invention are known to those skilled in the art. The term "pharmaceutically acceptable acid addition salt" refers to a salt formed with an inorganic acid or an organic acid that retains the bioavailability of the free base without other adverse effects. Inorganic acid salts include, but are not limited to, hydrochloride, hydrobromide, sulfate, nitrate, phosphate, and the like; organic acid salts include, but are not limited to, formate, acetate, 2,2-dichloroacetate, trifluoroacetate, propionate, caproate, caprylate, caprate, undecylenate, glycolate, gluconate, lactate, sebacate, adipate, glutarate, malonate, oxalate, maleate, succinate, fumarate, tartrate, citrate, palmitate, stearate, oleate, cinnamate, laurate, malate, glutamate, pyroglutamate, aspartate, benzoate, methanesulfonate, benzenesulfonate, p-toluenesulfonate, alginate, ascorbate, salicylate, 4-aminosalicylate, naphthalenedisulfonate, and the like. These salts may be prepared by methods known in this patent. The term "pharmaceutically acceptable base addition salts" refer to salts formed with inorganic or organic bases that retain the biological effectiveness of the free acid without other adverse effects. Salts derived from inorganic bases include, but are not limited to, sodium salts, potassium salts, lithium salts, ammonium salts, calcium salts, magnesium salts, iron salts, zinc salts, copper salts, manganese salts, aluminum salts, and the like. Preferred inorganic salts are ammonium salts, sodium salts, calcium salts and magnesium salts. Salts derived from organic bases include, but are not limited to, salts of: primary amines, secondary amines, and tertiary amines, substituted

amines, including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucosamine, theobromine, purine, piperazine, piperidine, N-ethylpiperidine, polyamine resins, and the like. Preferred organic bases include isopropylamine, diethylamine, ethanolamine, trimethylamine, dicyclohexylamine, choline, and hexocaffeine. These salts may be prepared by methods known in this patent.

In the present invention, unless otherwise specified, the term "ester" refers to esters derived from the compounds described herein, including physiologically hydrolyzable esters (which can be hydrolyzed under physiological conditions to release the compound of the present invention in free acid or alcohol form). The compounds of the present invention may also be esters themselves.

The compounds of the present invention may exist in the form of solvates (preferably hydrates), wherein the compounds of the present invention comprise a polar solvent, particularly such as water, methanol, or ethanol, as a structural element of the crystal lattice of the compounds. The amount of polar solvent, particularly water, may be present in a stoichiometric or non-stoichiometric ratio.

Those skilled in the art will understand that not all nitrogen heterocyclic rings are capable of forming N-oxides, as nitrogen requires an available lone pair of electrons to be oxidized into an oxide. Those skilled in the art will recognize nitrogen heterocyclic rings capable of forming N-oxides. Those skilled in the art will also recognize that tertiary amines are capable of forming N-oxides. The synthetic methods for preparing N-oxides of heterocyclic rings and tertiary amines are well-known to those skilled in the art, including oxidation of heterocyclic rings and tertiary amines using peroxy acids such as peroxyacetic acid and m-chloroperoxybenzoic acid (mCPBA), hydrogen peroxide, alkyl hydroperoxides such as tert-butyl hydroperoxide, sodium perborate, and dioxiranes such as dimethyldioxirane. These methods for preparing N-oxides have been extensively described and reviewed in the literature. See, for example: T.L. Gilchrist, Comprehensive Organic Synthesis, vol. 7, pp. 748-750 (A.R. Katritzky and A.J. Boulton, Eds., Academic Press); and G.W.H. Cheeseman and E.S.G Werstiuk, Advances in Heterocyclic Chemistry, vol. 22, pp. 390-392 (A.R. Katritzky and A.J. Boulton, Eds., Academic Press).

In the present invention, the term "metabolite" refers to a substance formed in the body when a compound of the present invention is administered. The metabolites of the compound may be identified using techniques well-established in the art, and their activity may be characterized through experimental methods. For example, such products may arise from oxidation, reduction, hydrolysis, amidation, deamidation, esterification, enzymolysis, and other reactions of the administered compound. Therefore, the present invention encompasses metabolites of the compounds of the present invention, including compounds produced by a method comprising contacting the compounds of the present invention with a mammal for a time sufficient to obtain their metabolites.

In the present invention, the term "prodrug" refers to certain derivatives of the compounds of the present invention that, when administered into or onto the body, can be converted, for example, by hydrolytic cleavage, into a compound of the present invention having the desired activity. Typically, such a prodrug will be a functional group derivative of the compound that is readily convertible in vivo to the desired therapeutically active compound. Additional information regarding the use of prodrugs may be found in Pro-drugs as Novel Delivery Systems, Volume 14, ACS Symposium Series (T. Higuchi and V. Stella). For example, the prodrug of the present invention may be prepared by substituting suitable functional groups present in the compounds of the present invention with certain moieties known to those skilled in the art as "pro-moieties" (e.g., as described in Design of Prodrugs by H. Bundgaard, Elsevier, 1985).

In the present application, the term "pharmaceutical composition" refers to a formulation of the compounds of the present invention with a medium generally accepted in the art for delivering biologically active compounds to mammals, such as humans. The medium comprises pharmaceutically acceptable carriers. An object of the pharmaceutical composition is to facilitate the administration to organisms, enhance the absorption of active ingredients, and thereby exert biological activity.

In the present application, the term "pharmaceutically acceptable carrier" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier approved by the relevant governmental regulatory authority or accepted for use in humans or domestic animals.

The terms "drug combination", "drug co-administration", "combination therapy", "administration of another therapy", "administration of another therapeutic agent", and the like, as used herein, refer to a therapeutic regimen obtained by mixing or combining more than one active ingredient, encompassing both fixed and non-fixed combinations of active ingredients. The term "fixed combination" refers to the simultaneous administration to a patient of at least one compound described herein and at least one synergistic agent in the form of a single entity or dosage form. The term "non-fixed combination" refers to the simultaneous administration, co-administration, or sequential administration at variable intervals of at least one compound described herein and at least one synergistic agent as separate entities to a patient. These principles are also applied in cocktail therapy, such as the administration of three or more active ingredients.

In the present invention, unless otherwise specified, the term "tumor" includes but is not limited to leukemia, gastrointestinal stromal tumor, histiocytic lymphoma, non-small cell lung cancer, small cell lung cancer, pancreatic cancer, lung squamous cell carcinoma, lung adenocarcinoma, breast cancer, prostate cancer, liver cancer, skin cancer, epithelial cell carcinoma, cervical cancer, ovarian cancer, colorectal cancer, nasopharyngeal cancer, brain cancer, bone cancer, esophageal cancer, melanoma, renal cancer, oral cancer, and other diseases.
In the present invention, unless otherwise specified, the term "treatment" refers to reversing, alleviating, inhibiting the progression of a disorder or condition to which such term is applied, or one or more symptoms thereof, or preventing such disorder or condition or one or more symptoms thereof.
Without departing from the common general knowledge in the art, the aforementioned preferred technical features may be freely combined in any manner, thereby yielding the various preferred embodiments of the present invention.

## Brief Description of Drawings

Figure 1 depicts the tumor volume changes in the MDA-MB-436 nude mice model following administration of Compounds **1** and **6.**
Figure 2 depicts the tumor volume changes in the MDA-MB-436 nude mice model following administration of Compounds **1, 6,** and **21.**
Figure 3 depicts the tumor volume changes in a gastric cancer PDX (patient-derived xenograft) model mice following combination therapy with Compound **6** and Carboplatin.
Figure 4 depicts the tumor volume changes in the SUM14PT nude mice subcutaneous xenograft model following combination therapy with Compound **6** and Carboplatin.

## Detailed Description

The scheme of the present invention will be explained below in conjunction with the embodiments. A person skilled in the art will understand that the following embodiments are intended to illustrate the invention only and should not be considered as limiting the scope of the invention. In case that specific technologies or conditions are not specified in the embodiments, technologies or conditions described in the literature in the art or the product specification shall prevail.
The reagents and raw materials used in the embodiments of the present invention are commercially available.

Table 1 Abbreviations and meanings thereof in the present invention

| | | | |
|---|---|---|---|
| AcOH | Acetic acid | HPLC | Liquid chromatography-mass spectrometry |
| Aq or aq | Aqueous solution | m/z | Mass/charge |
| BOC or Boc | Tert-butyloxycarbonyl | Me | Methyl |
| CPME | Cyclopentyl methyl ether | MeCN | Acetonitrile |
| DCE | 1,2-Dichloroethane | MeOH | Methanol |
| DABC O | 1,4-diazacyclo [2.2.2] octane | NMR | NMR |
| DCM | Methylene dichloride | THF | Tetrahydrofuran |
| DMA | N,N-dimethylacetamide | Sat or satd | Saturated |
| DMA P | 4-dimethylaminopyridine | rt | Room temperature |
| EA | Ethyl acetate | UV | Ultraviolet |
| PE | Petroleum ether | t-BuO H | Tert-butyl alcohol |
| DMF | N,N-dimethylformamide | Cbz | Benzyloxycarbonyl |
| TFA | Trifluoroacetic acid | mmol | Millimole |
| TEA | Triethylamine | mg | mg |
| DME | 1,2-dimethoxyethane | min | min |
| DMS O | Dimethyl sulfoxide | mL or ml | Milliliter |
| DPPF or dppf | 1,1’-bis(diphenylphosphino)fe rrocene | BINAP | 1,1’-binaphthyl-2,2’-bisdiphenylphos phine |

(continued)

| | | | |
|---|---|---|---|
| eq or equiv | Equivalent | *m-CPB* A | Metachloroperbenzoic acid |
| ESI | Electrospray ionization | TBS | Tert-butyldimethylsilane |
| Et | Ethyl | $Pd_2$(db a)$_3$ | Tris(dibenzylideneacetone)dipalladiu m |
| G | Gram | Xantph os | 4,5-bis(diphenylphosphino)-9,9-dimet hylxanthene |
| H | hours | NBS | N-bromosuccinimide |
| rt | Room temperature | TBAF | Tetrabutylammonium fluoride |

The structure of the compounds described in the present invention is determined by nuclear magnetic resonance (NMR) or mass spectrometry (MS). The NMR measurement was conducted using a Bruker AVANCE-400 spectrometer. The solvents used were deuterated dimethyl sulfoxide (DMSO-$d_6$), deuterated chloroform ($CDCl_3$), and deuterated methanol ($CD_3OD$), with tetramethylsilane (TMS) as the internal standard. The chemical shifts are reported in units of $10^{-6}$ (ppm).

Mass spectrometry (MS) was performed using an Agilent SQD (ESI) mass spectrometer (Manufacturer: Agilent, Signal: 6110).

HPLC analysis was performed using an Agilent 1200DAD High-pressure Liquid Chromatograph (SunFire C18, 150 × 4.6 mm, 5 wn, column) and a Waters 2695-2996 High-pressure Liquid Chromatograph (Gemini C18, 150 × 4.5 mm, 5 ym, column).

Thin-layer chromatography (TLC) was performed using Qingdao Haiyang GF254 silica gel plates. For analytical TLC, plates with a thickness of 0.15-0.2 mm were employed, while preparative thin-layer chromatography utilized silica gel plates with a thickness of 0.4-0.5 mm.

Column chromatography generally employs Qingdao Haiyang 100-200 or 200-300 mesh silica gel as the carrier.

Unless otherwise specified in the following embodiments, the reactions are all conducted under an argon atmosphere or a nitrogen atmosphere. An argon atmosphere or nitrogen atmosphere refers to a reaction flask connected to an argon or nitrogen balloon with a volume of approximately 1 L. A hydrogen atmosphere refers to a reaction flask connected to a hydrogen balloon with a volume of approximately 1 L. The hydrogenation reaction is typically performed by evacuating, filling with hydrogen, and repeating this process 3 times.

**Preparation of intermediates**

**Intermediate int-1: *N*-methyl-1',2',3',6'-tetrahydro-[3,4'-bipyridine]-6-carboxamide**

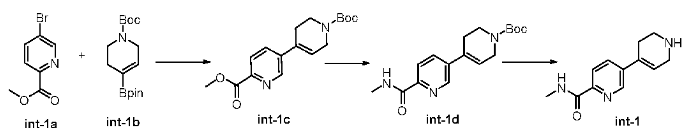

Step 1: The compound int-1a (1 g, 4.6 mmol), int-1b (1.7 g, 5.5 mmol), Pd(dppf)$Cl_2$ (0.3 g, 0.46 mmol) and potassium carbonate (1.6 g, 11.5 mmol) were added into a mixed solvent of 7 mL dioxane, 3 mL absolute ethyl alcohol and 4 mL water, then nitrogen was replaced for three times, and the mixture was reacted at 90°C under nitrogen protection for 2 h. After completion of the reaction was confirmed by TLC, the reaction mixture was cooled to room temperature, 30 mL dichloromethane and 20 mL water were added, and the layers were separated in a separatory funnel. The water phase was extracted twice with dichloromethane, the organic phases were combined, and then washed sequentially with water and saturated brine. The reaction liquid was dried with anhydrous sodium sulfate, filtered and concentrated by rotary evaporation. The obtained crude product was purified by column chromatography to obtain a compound int-1c (1g, white solid).

Step 2: The compound Int-1c (1 g, 3 mmol), aqueous methylamine aqueous solution (5 g, 161.3 mmol), and absolute methanol (20 mL) were added into a 100 mL reaction flask, and the mixture was stirred and reacted at room temperature overnight. After completion of the reaction was confirmed by TLC, the reaction liquid was concentrated under reduced pressure to dryness to obtain a compound int-1d (0.8g, white solid).

Step 3: The compound int-1d (0.5 g, 1.5 mmol) was added into 10 mL absolute methanol, followed by 10 mL solution of hydrochloric acid and dioxane (4 mol/L), and the mixture was stirred at room temperature for 0.5 - 1 h. After

completion of the reaction was confirmed by TLC, the reaction liquid was concentrated under reduced pressure to a dry compound int-1 (0.5 g, white solid).

**Embodiment 1:**

1'-(7-ethyl-6-oxo-5,6-dihydro-1,5-naphthyridin-3-yl)methyl)-N-methyl-1',2',3',6'-tetrahydro -[3,4'-bipyridine]-6-carboxamide

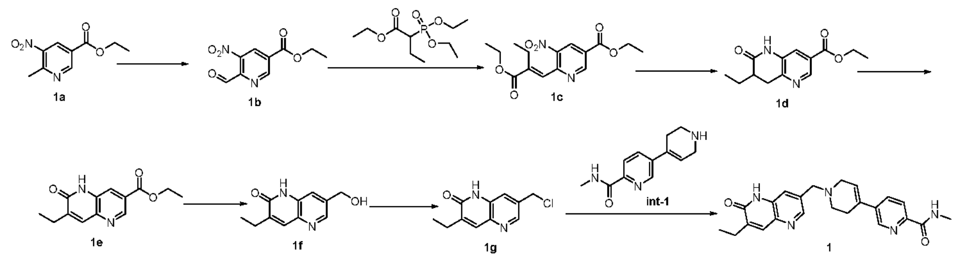


Step 1: The compound 1a (20 g, 95.1 mmol) and selenium dioxide (16 g, 144 mmol) were added into a 250 mL reaction flask. Then 120 mL 1,4-dioxane was added. The mixture was heated to 110°C and stirred for reaction overnight. After completion of the reaction was confirmed by TLC, the reaction liquid was filtered, and the filter residue was rinsed with ethyl acetate. The filtrates were combined and concentrated by rotary evaporation. The resulting crude product was purified by column chromatography to obtain a compound 1b (16 g, yellow solid). LC-MS: ESI $[M+H]^+$= 225.2.

Step 2: Sodium hydride (6.86g, 171.4mmol) was added into a 250ml reaction flask, followed by the addition of 60 mL 1,4-dioxane. Then, the nitrogen was replaced for three times, and cooled to 0°C, and a compound 2-triethyl phosphonobutanoate (43.2 g, 171.4 mmol) was slowly added dropwise under nitrogen protection. The reaction mixture was stirred at 0°C for 10 minutes, then heated to room temperature and stirred for another 10 minutes, followed by heated to 40°C and stirred for 5 minutes. The reaction mixture was cooled to -78°C. A compound INT1b (16 g, 71.4 mmol) was dissolved in 60 mL 1,4-dioxane, then slowly added dropwise, and the reaction mixture was stirred at -78°C for 1 hour. After completion of the reaction was confirmed by TLC, the reaction liquid was slowly added into an ice-cold saturated aqueous ammonium chloride aqueous solution to quench. The mixture was extracted three times with 150 mL ethyl acetate, dried with anhydrous sodium sulfate after combination of organic phases, filtered, and concentrated by rotary evaporation. The obtained crude product was purified by column chromatography to obtain a compound 1c (13.26g, brown liquid). LC-MS:ESI$[M+H]^+$=323.3.

Step 3: The compound 1c (13.26g, 41.1 mmol) was added into 100 mL absolute ethyl alcohol, followed by the addition of Pd/C (1.33g, 10%). The reaction mixture was stirred and reacted at room temperature overnight. After completion of the reaction was confirmed by LC-MS, the reaction liquid was filtered, the filter residue was rinsed extensively with ethanol, and the filtrates were combined and concentrated by rotary evaporation. A 4 mol/L hydrochloric acid solution was added in 1,4-dioxane (50 mL), the mixture was stirred at room temperature for 30 minutes, then ether was added to precipitate a large amount of solid. The solid was filtered and dried to obtain a compound 1d (7.32 g, white solid). LC-MS:ESI $[M+H]^+$ = 249.3; $^1H$ NMR (400 MHz, DMSO-$d_6$) δ 10.44 (s, 1H), 8.62 (d, *J* = 1.9 Hz, 1H), 7.75 (d, *J* = 1.9 Hz, 1H), 4.34 (q, *J* = 7.1 Hz, 2H), 3.87 (s, 0H), 3.24 (dd, *J* = 16.8, 6.3 Hz, 1H), 2.97 (dd, *J* = 16.8, 10.1 Hz, 1H), 1.81 - 1.65 (m, 1H), 1.52 - 1.36 (m, 1H), 1.32 (t, *J* = 7.1 Hz, 3H), 0.93 (t, *J* = 7.4 Hz, 3H).

Step 4: The compound 1d (7.32 g, 29.5 mmol) was added into a 250 mL reaction flask, followed by the addition of 120 mL 1,4-dioxane. Then, DDQ (7.38 g, 32.5 mmol) was added, and reflux was initiated. The mixture was allowed to react overnight. After completion of the reaction was confirmed by LC-MS, the reaction liquid was concentrated by rotary evaporation. A saturated sodium bicarbonate aqueous solution was added and stirred for 1 hour. The liquid was filtered and the filter residue was washed with water, followed by a small amount of ether. The residue was dried to obtain a compound 1e (2.31 g, yellow solid). LC-MS:ESI$[M+H]^+$=247.3.

Step 5: The compound 1e (2.0 g, 8.1 mmol) and 60 mL tetrahydrofuran were added into a 150mL reaction flask, cooled to 0°C, then a 2.5 mol/L tetrahydrofuran solution (6.48 mL, 16.2 mmol) of lithium aluminum hydride was added, and the mixture was reacted at 0°C for 2 hours. After completion of the reaction was confirmed by TLC, 5 mL water was added to quench the reaction, and then a large amount of anhydrous sodium sulfate was added to dry. The mixture was filtered, the filter residue was rinsed extensively with dichloromethane, and the filtrates were combined and concentrated by rotary evaporation. The residue was dried to obtain a compound 1f (1.2 g, white solid). LC-MS:ESI $[M+H]^+$ = 205.3; $^1H$ NMR (400 MHz, DMSO-$d_6$) δ 11.87 (s, 1H), 8.03 (d, *J* = 2.0 Hz, 1H), 7.36 (d, *J* = 1.0 Hz, 1H), 7.34 (dd, *J* = 2.0, 0.9 Hz, 1H), 4.51 (s, 2H), 2.52 (d, *J* = 1.8 Hz, 1H), 1.15 (t, *J* = 7.4 Hz, 3H).

Step 6: The compound 1f (0.82 g, 4.0 mmol) was added into a 50 mL reaction flask, and then 20 mL dichloromethane and 1 mL N,N-dimethylformamide were added. The mixture was cooled to 0 °C, and then thionyl chloride (0.87 mL, 12 mmol) was added dropwise. The mixture was reacted at 0 °C for 1 hour. After completion of the reaction was confirmed by TLC, the reaction liquid was concentrated by rotary evaporation. The crude product was purified by column chromatography to obtain a compound 1g (0.66 g, gray solid). LC-MS:ESI[M+H]$^+$=223.7 $^1$H NMR (400 MHz, DMSO) δ 12.16 (s, 1 H), 8.55 (s, 1 H), 7.97 - 7.73 (m, 2 H), 4.95 (s, 2 H), 2.56 (d, *J* = 7.4 Hz, 2 H), 1.19 (t, *J* = 7.4 Hz, 3 H).

Step 7: The compound int-1 (0.05 g, 0.23 mmol), 1g (0.06 g, 0.28 mmol), N,N-diisopropylethylamine (0.15 g, 1.15 mmol), and potassium iodide (0.19 g, 1.15 mmol) were added into 10 mL anhydrous acetonitrile. The mixture was stirred at 80 °C for 2 hours. After completion of the reaction was confirmed by TLC, the reaction liquid was concentrated under reduced pressure. The crude product was purified by column chromatography to obtain a compound 1 (0.02 g, white solid); LC-MS: ESI [M+H]$^+$=404.5;$^1$H NMR (400 MHz, DMSO): 11.85 (s, 1 H), 8.71 (d, *J* = 5.0 Hz, 1 H), 8.69 (s, 1 H), 8.41 (d, *J* = 1.3 Hz, 1 H), 8.06 - 7.91 (m, 2 H), 7.75 (s, 1 H), 7.64 (s, 1 H), 6.42 (s, 1 H), 3.72 (s, 2 H), 3.16 (s, 2 H), 2.81 (d, *J* = 4.8 Hz, 3 H), 2.70 (s, 2 H), 2.54 (d, *J* = 7.4 Hz, 4 H), 1.18 (t, *J* = 7.4 Hz, 3 H).

**Embodiment 2:**

1'-((7-ethyl-6-oxo-5,6-dihydro-1,5-naphthyridin-3-yl)methyl)-N-(methyl-d3)-1',2',3',6'-tetrahy dro-[3,4'-bipyridine]-6-carboxamide

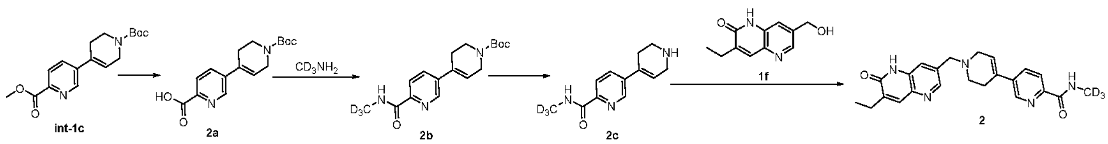


int-1c 2a 2b 2c 2

Step 1: The compound int-1c (1.4g, 4.5 mmol) was dissolved in 20 mL MeOH. 5 mL water was added, followed by the addition of lithium hydrate (570 mg, 13.5 mmol). The mixture was reacted at room temperature for 12 hours, with the progress monitored by TLC. Upon the completion of the reaction, the pH was adjusted to 6 by adding 2M HCl. The mixture was extracted with EA (3 × 25 mL) and dried with anhydrous sodium sulfate after combination of organic phases, and concentrated under vacuum by rotary evaporation to obtain a product 2a (460 mg, pale yellow solid).

Step 2: The compound 2a (230 mg, 0.7 mmol), EDCI (191 mg, 1 mmol), HOBT (100 mg, 1 mmol), and 2 mL DMF were added, followed by N-methylmorpholine (250 mg, 2.8 mmol) and d3-methylamine hydrochloride (49 mg, 0.7 mmol). The mixture was reacted at room temperature for 12 hours, with the progress monitored by TLC. After the starting material was completely consumed, the mixture was diluted with water, extracted with EA, and the organic phase was washed with water five times, dried with anhydrous sodium sulfate, and concentrated by rotary evaporation to obtain a crude product 2b (150 mg, pale yellow oily liquid).

Step 3: The crude product 2b (220 mg, 0.7 mmol) was dissolved in 4 mL MeOH, and then 1.1 mL 4M HCl was added in a dioxane solution. The mixture was reacted at room temperature for 12 hours, with the progress monitored by TLC. Upon completion of the reaction, potassium carbonate was added and stirred for 30 minutes, and then filtered to remove the potassium carbonate, obtaining the crude product of compound 2c (270 mg, yellow-green solid).

Step 4: The compound 1f (40.6 mg, 0.2 mmol), 1 mL DCM, and DMF (15 mg, 0.02 mmol) were added, followed by the addition of thionyl chloride (70 mg, 0.6 mmol) at 0°C. The mixture was reacted for 30 minutes, and then continued at room temperature for another 30 minutes, with the progress monitored by TLC. After complete consumption of the starting material, the mixture was concentrated under vacuum by rotary evaporation. Subsequently, 2c (44.2 mg, 0.2 mmol) and DIPEA (180 mg, 1.4 mmol) were added. KI (10 mg, 0.06 mmol) was added, followed by 2 mL acetonitrile. The mixture was heated to 80 °C and the mixture was reacted for 2 hours, with the progress monitored by TLC. Upon completion, 2 mL saturated sodium bicarbonate solution was added, extracted with EA (2 × 3 mL), the organic phases were combined, and purified by preparative TLC to obtain 2 (12 mg, light yellow solid). LC-MS:ESI[M+H]$^+$=407.5 $^1$H NMR (400 MHz, DMSO): 11.85 (s, 1 H), 8.71 (d, *J* = 5.0 Hz, 1 H), 8.69 (s, 1 H), 8.41 (d, *J* = 1.3 Hz, 1 H), 8.06 - 7.91 (m, 2 H), 7.75 (s, 1 H), 7.64 (s, 1 H), 6.42 (s, 1 H), 3.72 (s, 2 H), 3.16 (s, 2 H), 2.70 (s, 2 H), 2.54 (d, *J* = 7.4 Hz, 4 H), 1.18 (t, *J* = 7.4 Hz, 3 H).

**Embodiment 3:**

1'-(7-ethyl-6-oxo-5,6-dihydro-1,5-naphthyridin-3-yl)methyl-*d*)-N-methyl-1',2',3',6'-tetrahydro -[3,4'-bipyridine]-6-carboxamide

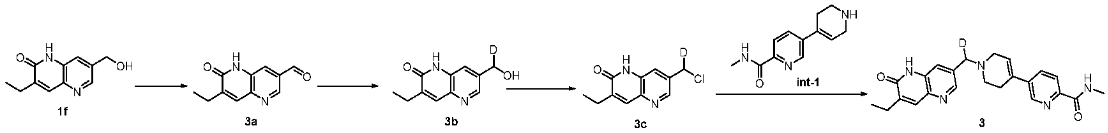


Step 1: The compound 1f (0.4 g, 2.0 mmol) and dichloromethane (20 mL) were added into a 50 mL reaction flask, and cooled to 0 °C, and then Dess-Martin periodinane (1.7 g, 4.0 mmol) was added. The mixture was reacted at 0 °C for 1.5 hours. After completion of the reaction was confirmed by TLC, the mixture was filtered through diatomaceous earth, the filter cake was rinsed with dichloromethane, the filtrate was concentrated by rotary evaporation, and purified by column chromatography to obtain a compound 3a (0.38 g, light yellow solid). LC-MS:ESI$[M+H]^+$=203.2; $^1$H NMR (400 MHz, DMSO-$d_6$) δ 12.17 (s, 1H), 10.16 (s, 1H), 8.92 (d, *Jb* = 1.8 Hz, 1H), 7.85 - 7.83 (m, 2H), 2.59 (qd, *J* = 7.4, 1.3 Hz, 2H), 1.21 (t, *J* = 7.4 Hz, 3H).

Step 2: The compound 3a (0.2 g, 1 mmol) was added into a reaction flask, 15 mL ether and 4 mL methanol were added and cooled to 0°C, 46 mg (1.1 mmol) sodium borodeuteride was added, and the mixture was reacted at room temperature for 1 hour. After completion of the reaction was confirmed by TLC, the mixture was quenched with a small amount of water, concentrated by rotary evaporation, and a small amount of ice-water was added to slurry, filter, and dry to obtain a compound 3b (110 mg, white solid); LC-MS: ESI $[M+H]^+$ = 206.2.

Step 3: The compound 3b (110 mg, 0.5 mmol) was added into a 25 mL reaction flask, and then 10 mL dichloromethane and 0.1 mL N,N-dimethylformamide were added. The mixture was cooled to 0 °C, and then thionyl chloride (88 mg, 0.75 mmol) was added dropwise. The mixture was reacted at 0 °C for 1 hour. After completion of the reaction was confirmed by TLC, the reaction liquid was concentrated by rotary evaporation. The obtained crude product by was purified column chromatography to obtain a compound 3c (103 mg, white solid); LC-MS: ESI $[M+H]^+$ = 224.7.

Step 4: The compound 3c (67 mg, 0.3 mmol), int-1 (96 mg, 0.33 mmol), N,N-diisopropylethylamine (194 mg, 1.5 mmol), and potassium iodide (5 mg, 0.03 mmol) were added into 10 mL anhydrous acetonitrile and stirred at 80 °C for 2 hours. After completion of the reaction was confirmed by TLC, the reaction liquid was concentrated under reduced pressure, and the obtained crude product was purified by column chromatography to obtain a compound **3** (60 mg, light yellow solid); LC-MS: ESI $[M+H]^+$ = 405.5; 1 H NMR (400 MHz, DMSO) δ 11.85 (s, 1 H), 8.70 (s, 2 H), 8.42 (d, *J* = 1.7 Hz, 1 H), 7.99 (d, J = 2.0 Hz, 2 H), 7.76 (s, 1 H), 7.65 (s, 1 H), 6.43 (s, 1 H), 3.69 (s, 1 H), 3.17 (s, 2 H), 2.82 (d, *J* = 4.8 Hz, 3 H), 2.71 (s, 2 H), 2.60-2.53 (m, 4 H), 1.19 (t, *J* = 7.4 Hz, 3 H).

**Embodiment 4:**

1'-((2-Ethyl-5-fluoro-3-oxo-3,4-dihydroquinoxalin-6-yl)methyl)-N-(methyl-$d_3$)-1',2',3',6'-tetra hydro-[3,4'-bipyridine]-6-carboxamide

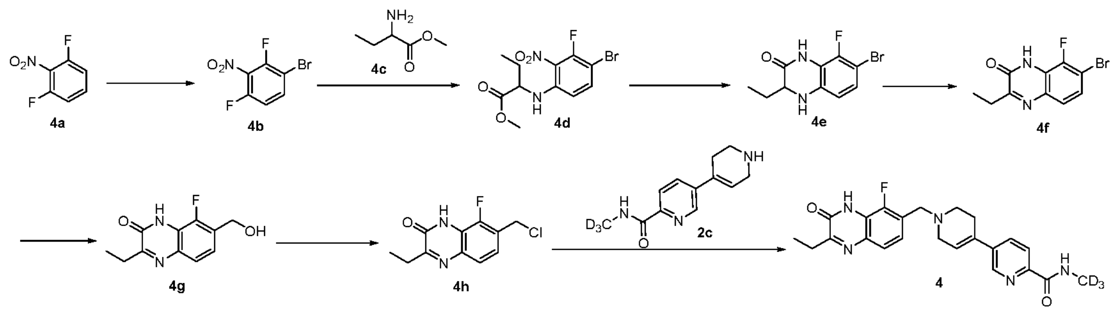


Step 1: The compound 4a (50 g, 314 mmol) and N-bromosuccinimide (67 g, 377 mmol) were added into a reaction flask, 300 mL sulfuric acid was added and heated to 80 °C, and the mixture was reacted overnight. After completion of the reaction was confirmed by TLC, the reaction liquid was cooled to room temperature, and then the liquid was slowly added into ice water to dilute, and was extracted three times with ethyl acetate, and washed sequentially with water and a saturated aqueous solution of sodium bicarbonate after combination of organic phases, dried with anhydrous sodium

sulfate, filtered, and concentrated by rotary evaporation. The obtained crude product was purified by column chromatography to obtain a compound 4b (65 g, light yellow solid); LC-MS: ESI $[M+H]^+$ = 238.9.

Step 2: The compound 4b (43g, 181mmol) and a compound 4c (21g, 181mmol), N,N-diisopropylethylamine (70g, 543mmol), and N,N-dimethylformamide (100ml) were added into a 500ml reaction flask, and the mixture was stirred and reacted at room temperature overnight. After completion of the reaction was confirmed by TLC, the reaction liquid was concentrated under reduced pressure, extracted with ethyl acetate and water for three times, dried with anhydrous sodium sulfate after combination of organic phases, filtered, and concentrated by rotary evaporation. The obtained crude product was purified by column chromatography to obtain a compound 4d (37 g, orange-red solid); LC-MS: ESI $[M+H]^+$ = 336.1.

Step 3: The compound 4d (26 g, 78 mmol) was added into a mixture of 200 mL absolute methanol and 5 mL water, and then ammonium chloride (35 g, 621 mmol) was added. The mixture was cooled to 0 °C, and zinc powder (43 g, 621 mmol) was slowly added. The mixture was heated to room temperature, and the mixture was reacted at room temperature for 1 hour. After completion of the reaction was confirmed by TLC, the filtrate was filtered and concentrated under reduced pressure. Subsequently, a dioxane solution (30 mL) containing 4 mol hydrochloric acid was added, and the mixture was reacted at room temperature for 1 hour. After completion of the reaction was confirmed by TLC, the petroleum ether was added to precipitate the solid. After filtering and drying, a compound 4e (16 g, off-white solid) was obtained; LC-MS: ESI $[M+H]^+$ = 274.1.

Step 4: The compound 4e (16 g, 57 mmol) and 2,3-dichloro-5,6-dicyanobenzoquinone (16 g, 69 mmol) were added into 500 mL dichloromethane and the mixture was reacted overnight at room temperature. After completion of the reaction was confirmed by TLC, the reaction liquid was concentrated under reduced pressure, quenched with a saturated aqueous solution of sodium bicarbonate, extracted with ethyl acetate for three times, dried with anhydrous sodium sulfate after completion of organic phases, filtered, and concentrated by rotary evaporation. The obtained crude product was purified by column chromatography to obtain a compound 4f (7.9 g, brown solid); LC-MS: ESI $[M+H]^+$ = 272.1.

Step 5: The compound 4f (0.5 g, 1.8 mmol), tributylstannyl methanol (0.65 g, 2.0 mmol), and Xphos-Pd-$G_2$ (73 mg, 0.09 mmol) were added, 15 mL dioxane was added, purged with nitrogen, heated to 80°C and then the mixture was reacted at 80°C overnight. After completion of the reaction was confirmed by TLC, the reaction liquid was added with water, extracted with ethyl acetate for three times, dried with anhydrous sodium sulfate after combination of organic phases, filtered, and concentrated by rotary evaporation. The obtained crude product was purified by column chromatography to obtain a compound 4g (0.4 g, light yellow solid); LC-MS: ESI $[M+H]^+$ = 223.2.

Step 6: The compound 4g (0.4 g, 0.8 mmol) was added into 10 mL aqueous hydrobromic acid, heated to 80°C, and the mixture was reacted for 3 hours. After completion of the reaction was confirmed by TLC, the reaction liquid was quench withed a saturated aqueous solution of sodium bicarbonate, extracted with dichloromethane for three times, dried with anhydrous sodium sulfate after combination of organic phases, filtered, and concentrated by rotary evaporation. The obtained crude product was purified by column chromatography to obtain a compound 4h (0.24 g, light yellow solid); LC-MS: ESI $[M+H]^+$ = 286.1.

Step 7: The compound 4h (57 mg, 0.2 mmol), 2c (64 mg, 0.22 mmol), N,N-Diisopropylethylamine (0.13 g, 1.0 mmol) and potassium iodide (3 mg, 0.02 mmol) were added into 10 mL anhydrous acetonitrile, and stirred at 85°C for 2 h. After completion of the reaction was confirmed by TLC, the reaction liquid was concentrated under reduced pressure to a crude product, and the crude product was purified by column chromatography to obtain a compound **4** (16 mg, faint yellow solid). LC-MS:ESI$[M+H]^+$=425.2; $^1$H NMR (400 MHz, DMSO) δ 12.42 (s, 1 H), 8.75 - 8.63 (m, 2 H), 8.03 - 7.91 (m, 2 H), 7.55 (d, *J* = 8.3 Hz, 1 H), 7.36 - 7.25 (m,1 H), 6.41 (s,1 H), 3.76 (s, 2 H), 2.89 - 2.76 (m, 4 H), 2.72 (t, *J*=5.5 Hz, 2 H), 2.54 (s, 2 H), 1.22 (s, 3 H).

**Embodiment 5:**

*N*-Cyclopropyl-1'-[(7-ethyl-6-oxo-5,6-dihydro-1,5-naphthyridin-3-yl)methyl-d]-1',2',3',6'-tetra hydro-[3,4'-bipyridine]-6-carboxamide

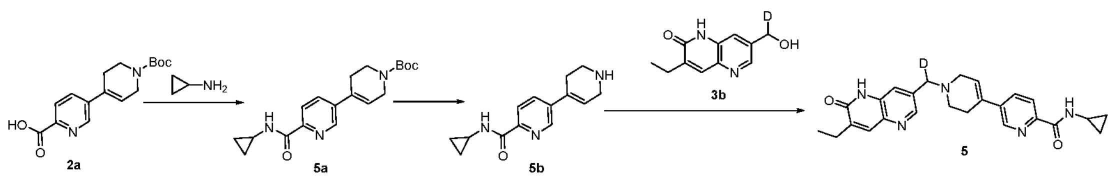

Step 1: The compound 2a (180 mg, 0.6 mmol), EDCI (144 mg, 0.75 mmol), HOBT (100 mg, 0.75 mmol), and 2 mL DMF were added, followed by N-methylmorpholine (290 mg, 3.2 mmol) and cyclopropylamine (34 mg, 0.6 mmol). The

mixture was reacted at room temperature for 12 hours, with the progress monitored by TLC. After complete consumption of the starting material, the reaction liquid was diluted with water, extracted with EA, washed with water for the organic phases five times, dried over anhydrous sodium sulfate, and concentrated by rotary evaporation to obtain a crude product 5a (180 mg, yellow oily liquid).

Step 2: The crude product of 5a (180 mg, 0.6 mmol) was dissolved in 5 mL MeOH, and then 0.9 mL 4M HCl was added in dioxane solution. The mixture was reacted at room temperature for 12 hours, with the progress monitored by TLC. After the reaction was completed, potassium carbonate was added, stirred for 30 minutes, and then filtered to remove the potassium carbonate to obtain a crude product of compound 5b (180 mg, yellowish-brown solid).

Step 3: The compound 3b (40.6 mg, 0.2 mmol), 1 mL DCM, and DMF (15 mg, 0.02 mmol) were added, and then thionyl chloride (70 mg, 0.6 mmol) was added at 0 °C. The mixture was reacted at 0 °C for 30 minutes, and then continued at room temperature for another 30 minutes, with the progress monitored by TLC. After the starting material was completely consumed, the reaction liquid was concentrated under vacuum by rotary evaporation, then 5b (44.2 mg, 0.2 mmol) and DIPEA (180 mg, 1.4 mmol) were added. KI (10 mg, 0.06 mmol) was added, followed by 2 mL acetonitrile, heated to 80 °C and the mixture was reacted for 2 hours, with the progress monitored by TLC. After the reaction was completed, 2 mL saturated sodium bicarbonate solution was added, and then extracted with EA (2x3 mL). The organic phases were combined and the reaction liquid was purified by TLC to obtain 5 (8 mg, light yellow solid). LC-MS:ESI$[M+H]^+$=431.2 $^1$HNMR (400 MHz, DMSO) δ 11.87 (s, 1 H), 8.69 - 8.65 (m, 2 H), 8.43 (s, 1 H), 8.04 - 7.94 (m, 2 H), 7.76 (s, 1 H), 7.66 (s, 1 H), 6.41 (s, 1 H), 3.76 - 3.66 (m, 1 H), 3.17 (s, 2 H), 2.95 - 2.85 (m, 1 H), 2.77 - 2.68 (m, 3 H), 2.55 (q, *J* = 13.2 Hz, 2 H), 2.36 - 2.33 (m, 1 H), 1.19 (t, *J* = 13.2 Hz, 3 H), 0.75 - 0.61 (m, 4 H).

**Embodiment 6:**

1'-((7-(ethyl-d5)-6-oxo-5,6-dihydro-1,5-naphthyridin-3-yl)methyl)-N-methyl-1',2',3',6'-tetrahy dro-[3,4'-bipyridine]-6-carboxamide

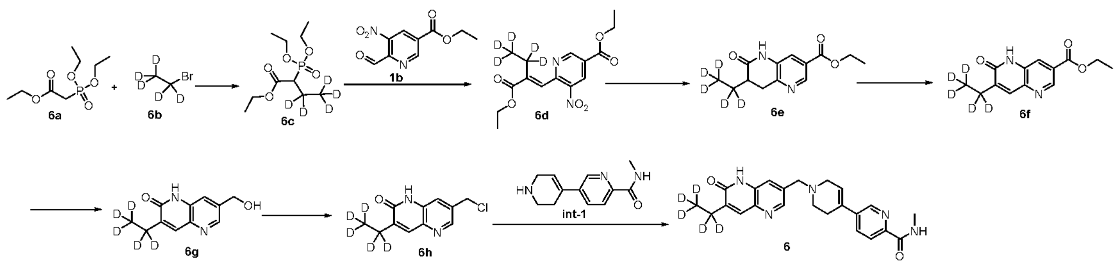

Step 1: 60% sodium hydride (16 g, 406 mmol) was added into DME (200 mL), purged with nitrogen for three times, cooled to 0 °C, and a compound 6a (76 g, 338 mmol) was added dropwise. The mixture was reacted at room temperature for 2 hours, and then the deuteromethyl bromide (50 g, 439 mmol) was added dropwise. The reaction liquid was heated to 60 °C and the mixture was reacted at 60 °C for 3 hours. After the reaction progress was monitored by HPLC, the reaction liquid was slowly poured into iced water to quench the reaction, extracted with ethyl acetate for three times, dried with anhydrous sodium sulfate after combination of organic phases, filtered, and concentrated by rotary evaporation. Then a crude product compound 6c (83 g, colorless liquid) was obtained.

Step 2: 60 % sodium hydride (13 g, 322 mmol) was added into the reaction flask, and then 200 mL tetrahydrofuran was added. Afterwards, the mixture was purged with nitrogen for three times, and cooled to 0°C and a compound 6c (83 g, 322 mmol) was added slowly dropwise, stirred and reacted at 0°C for 10 minutes. Then, the reaction liquid was heated to room temperature, and stirred. The mixture was reacted at room temperature for additional 10 minutes. The reaction liquid was further heated to 40 °C, and stirred and finally cooled to -78 °C after reaction for 5 minutes. The solution of compound 1b (48 g, 215 mmol) dissolved in 200 mL tetrahydrofuran was slowly added dropwise, stirred and reacted at -78 °C for 1 hour. After completion of the reaction was confirmed by TLC, the reaction liquid was slowly quenched by adding it to an iced saturated aqueous solution of ammonium chloride, extracted for three times with ethyl acetate, dried with anhydrous sodium sulfate after combination of organic phases, filtered, and concentrated by rotary evaporation. The obtained crude product was purified by column chromatography to obtain a compound 6d (41 g, yellow-green liquid). LC-MS:ESI $[M+H]^+$=328.4.

Step 3: The compound 6d (1.0 g, 3.0 mmol) was added into 10 mL absolute ethyl alcohol, and then Pd/C (0.1 g, 10 %) was added. The mixture was purged with hydrogen for three times, stirred and reacted at room temperature overnight. After completion of the reaction was confirmed by LC-MS, the reaction liquid was filtered, the filter residue was rinsed

extensively with ethanol, and the filtrates were combined, and concentrated by rotary evaporation. The solution of 1,4-dioxane containing 4 mol/L hydrochloric acid (12 mL) was added, and stirred at room temperature for 30 minutes. Diethyl ether was added to precipitate a large amount of solids. The solids were filtered and dried to obtain a compound 6e (0.51 g, white solid); LC-MS: ESI $[M+H]^+$ = 254.3.

Step 4: The compound 6e (0.51 g, 2.0 mmol) was added into a 50 mL reaction flask, followed by the addition of 15 mL 1,4-dioxane. Then, DDQ (0.50 g, 2.2 mmol) was added, and reflux was initiated. The mixture was allowed to react overnight. After completion of the reaction was confirmed by LC-MS, the reaction liquid was concentrated by rotary evaporation. A saturated aqueous solution of sodium bicarbonate was added and stirred for 1 hour. The liquid was filtered and the residue was washed with water, and then rinsed with a small amount of ether. The residue was dried to obtain a compound 6f (0.3 g, yellow solid). LC-MS:ESI$[M+H]^+$=252.3.

Step 5: The compound 6f (0.3 g, 1.2 mmol) and 15 mL tetrahydrofuran were added into a reaction flask, cooled to 0°C, and then a 2.5 mol/L tetrahydrofuran solution (1.44 mL, 3.6 mmol) of lithium aluminum hydride was added. The mixture was reacted at 0°C for 2 hours. After completion of the reaction was confirmed by TLC, 1 mL water was added to quench the reaction. A large amount of anhydrous sodium sulfate was added for drying, and then filtered. The filter residue was rinsed with a large amount of dichloromethane. After the filtrates were combined, the residue was concentrated by rotary evaporation and then dried to obtain compound 6g (0.2 g, yellow solid). LC-MS:ESI$[M+H]^+$=210.3.

Step 6: The compound 6g (0.2 g, 0.96 mmol) was added into a 25 mL reaction flask, and then 10 mL dichloromethane and 0.1 mL N,N-dimethylformamide were added. The mixture was cooled to 0 °C, and then thionyl chloride (0.35 g, 2.9 mmol) was added dropwise. The mixture was reacted at 0 °C for 1 hour. After completion of the reaction was confirmed by TLC, the reaction liquid was concentrated by rotary evaporation. The obtained crude product was purified by column chromatography to obtain a compound 6h (0.2 g, gray solid). LC-MS:ESI$[M+H]^+$=228.7.

Step 7: The compound 6h (0.2 g, 0.88 mmol), int-1 (0.25 g, 0.88 mmol), N,N-diisopropylethylamine (0.45 g, 3.52 mmol), and potassium iodide (15 mg, 0.09 mmol) were added into 10 mL anhydrous acetonitrile. The mixture was stirred at 85 °C for 2 hours. After completion of the reaction was confirmed by TLC, the reaction liquid was concentrated under reduced pressure. The obtained crude product was purified by column chromatography to obtain a compound **6** (160 mg, white solid) LC-MS:ESI$[M+H]^+$=409.5; $^1$H NMR (400 MHz, $CDCl_3$) δ 10.97 (s, 1H), 8.57 (d, *J* = 2.0 Hz, 1 H), 8.54 (d, *J* = 1.7 Hz, 1 H), 8.15 (d, *J*= 8.2 Hz, 1 H), 7.96 (d, *J* = 4.9 Hz, 1 H), 7.86 (s, 1 H), 7.80 (dd, *J* = 8.2, 2.3 Hz, 1 H), 7.66 (s, 1 H), 6.23 (s, 1 H), 3.78 (s, 2 H), 3.27 (d, *J* = 3.1 Hz, 2 H), 3.04 (d, *J* = 5.1 Hz, 3 H), 2.79 (t, *J* = 5.6 Hz, 2 H), 2.60 (d, *J* = 1.4 Hz, 2 H).

## Embodiment 7:

1'-((7-(ethyl-$d_5$)-6-oxo-5,6-dihydro-1,5-naphthyridin-3-yl)methyl)-N,2-dimethyl-1',2',3',6'-tetr ahydro-[3,4'-bipyridine]-6-carboxamide

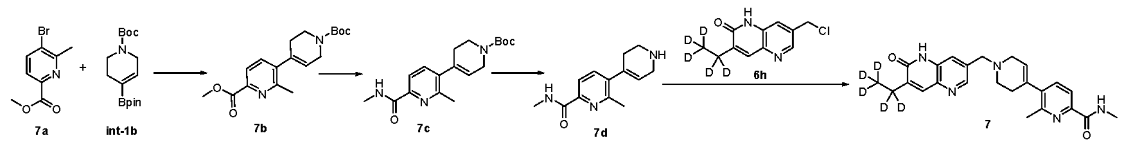

Step 1: The compound 7a (0.5 g, 2.2 mmol), int-1b (2.4 g, 5.5 mmol), $Pd(dppf)Cl_2$ (0.16 g, 0.22 mmol) and potassium carbonate (0.7 g, 5.5 mmol) were added into a mixed solvent of 7 mL dioxane, 3 mL absolute ethyl alcohol and 4 mL water, then the mixture was purged with nitrogen for three times, and the mixture was reacted at 90°C under nitrogen protection for 2 h. After completion of the reaction was confirmed by TLC, the reaction mixture was cooled to room temperature, 30 mL dichloromethane and 20 mL water were added, and the layers were separated in a separatory funnel. The water phase was extracted twice with dichloromethane, then washed sequentially with water and saturated brine, dried with anhydrous sodium sulfate after combination of organic phases, filtered, and concentrated by rotary evaporation. The obtained crude product was purified by column chromatography to obtain a compound 7b (0.7 g, white solid).

Step 2: The compound 7b (0.7 g, 26 mmol) was added into 10 mL absolute methanol, followed by 20 mL aqueous solution of methylamine. The mixture was stirred and reacted at room temperature overnight. After completion of the reaction was confirmed by TLC, the reaction liquid was concentrated under reduced pressure to a dry compound 7c (0.5 g, white solid).

Step 3: The compound 7c (0.5 g, 1.7 mmol) was added into 10 mL absolute methanol, followed by 10 mL solution of hydrochloric acid and dioxane (4 mol/L), and the mixture was stirred at room temperature for 0.5 - 1 h. After completion of the reaction was confirmed by TLC, the reaction liquid was concentrated under reduced pressure to a dry compound 7d (0.3 g, white solid).

Step 4: The compound 7d (0.05 g, 0.25 mmol), 6h (0.071 g, 0.231 mmol), N,N-diisopropylethylamine (0.17 g, 1.25 mmol) and potassium iodide (0.22 g, 1.25 mmol) were added into 10 mL anhydrous acetonitrile, and the mixture was stirred at 80°C for 2 h. After completion of the reaction was confirmed by TLC, the reaction liquid was concentrated under reduced pressure to a crude product, and the obtained crude product was purified by column chromatography to obtain a compound 7 (0.024 g, white solid). LC-MS:ESI$[M+H]^+$=423.2. $^1$H NMR (400 MHz, Chloroform-d) δ 11.90 (s, 1H), 8.50 (d, *J* = 2.0 Hz, 1H), 7.99 (d, *J* = 5.0 Hz, 1H), 7.91 (d, *J* = 7.8 Hz, 1H), 7.81 (s, 1H), 7.67 (d, *J* = 1.9 Hz, 1H), 7.46 (d, *J* = 7.8 Hz, 1H), 5.64-5.50 (m, 1H), 3.73 (s, 2H), 3.15 (d, *J* = 3.1 Hz, 2H), 2.96 (d, *J* = 5.1 Hz, 3H), 2.69 (t, *J* = 5.5 Hz, 2H), 2.47 (s, 3H), 2.36-2.32 (m, 2H).

## Embodiment 8:

1'-((7-ethyl-6-oxo-5,6-dihydro-1,5-naphthyridin-3-yl)methyl)-2-methyl-N-(methyl-$d_3$)-1',2',3', 6'-tetrahydro-[3,4'-bipyridine]-6-carboxamide

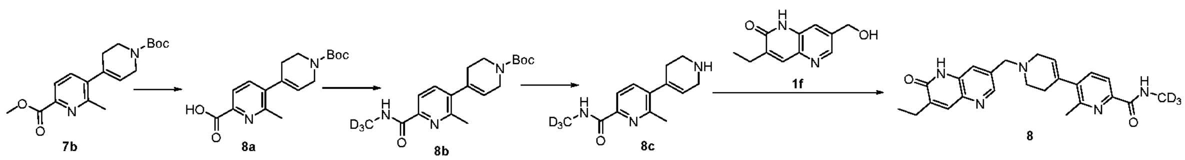

7b 8a 8b 8c 8

The compound 1'-((7-ethyl-6-oxo-5,6-dihydro-1,5-naphthyridin-3-yl)methyl)-2-methyl-N-(methyl-$d_3$)-1',2',3',6'-tetrahydro-[3,4'-bipyridine]-6-carboxamide was obtained via a similar procedure as that described for Embodiment 2. LC-MS:ESI$[M+H]^+$=421.2. $^1$H NMR (400 MHz, DMSO-$d_6$) δ 11.86 (s, 1H), 8.56 (s, 1H), 8.43 (d, *J* = 1.9 Hz, 1H), 7.80 (d, *J* = 7.8 Hz, 1H), 7.76 (s, 1H), 7.70-7.60 (m, 2H), 5.70 (s, 1H), 3.73 (s, 2H), 3.12 (d, J = 3.1 Hz, 2H), 2.69 (t, *J* = 5.5 Hz, 2H), 2.57-2.53 (m, 5H), 2.36 (s, 2H), 1.18 (t, *J* = 7.4 Hz, 3H).

## Embodiment 9:

1'-((7-(ethyl-$d_5$)-6-oxo-5,6-dihydro-1,5-naphthyridin-3-yl)methyl)-2-fluoro-N-methyl-1',2',3', 6'-tetrahydro-[3,4'-bipyridine]-6-carboxamide

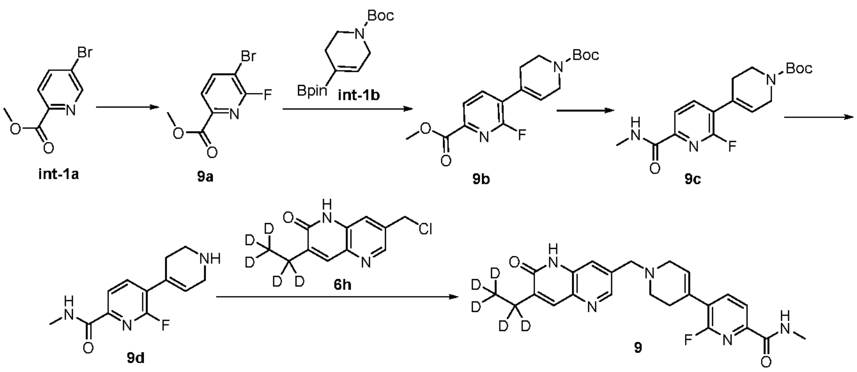

int-1a 9a 9b 9c 9d 9

Step 1: The compound int-1a (3.7 g, 17.1 mmol) and silver fluoride (10 g, 68.4 mmol) were added into 40 mL anhydrous acetonitrile, then the nitrogen was replaced for three times. The mixture was stirred at room temperature for 48 h under nitrogen protection. After completion of the reaction was confirmed by TLC, the reaction liquid was concentrated to obtain a dry crude product, and the crude product was purified by column chromatography to obtain a compound 9a (2.2 g, white solid).

Step 2: The compound 9a (1 g, 4.3 mmol), int-1b (1.59 g, 5.1 mmol), Pd(dppf)$Cl_2$ (0.31 g, 0.43 mmol) and potassium carbonate (1.5 g, 10.8 mmol) were added into a mixed solvent of 7 mL dioxane, 3 mL absolute ethyl alcohol and 4 mL water, then nitrogen was replaced for three times, and the mixture was reacted at 90°C under nitrogen protection for 2 h. After completion of the reaction was confirmed by TLC, the reaction mixture was cooled to room temperature, 30 mL dichloromethane and 20 mL water were added, and the layers were separated in a separatory funnel. The water phase

was extracted twice with dichloromethane, then washed sequentially with water and saturated brine, dried with anhydrous sodium sulfate after combination of organic phases, filtered, and concentrated by rotary evaporation. The obtained crude product was purified by column chromatography to obtain a compound 9b (1.2 g, white solid).

Step 3: The compound 9b (1 g, 2.9 mmol), an aqueous solution of methylamine (5 g, 161.3 mmol) and absolute methanol (20 mL) were added into a 100 mL reaction flask, and the mixture was stirred and reacted at room temperature overnight. After completion of the reaction was confirmed by TLC, the reaction liquid was concentrated under reduced pressure to a dry compound 9c (0.8 g, white solid).

Step 4: The compound 9c (0.5 g, 1.4 mmol) was added into 10 mL absolute methanol, followed by 10 mL solution of hydrochloric acid and dioxane (4 mol/L), and the mixture was stirred at room temperature for 0.5 - 1 h. After completion of the reaction was confirmed by TLC, the reaction liquid under reduced pressure was concentrated to a dry compound 9d (0.3 g, white solid).

Step 5: The compound 9d (0.05 g, 0.21 mmol), 6h (0.056 g, 0.26 mmol), N,N-diisopropylethylamine (0.14 g, 1.05 mmol) and potassium iodide (0.17 g, 1.05 mmol) were added into 10 mL anhydrous acetonitrile, and the mixture was stirred at 80°C for 2 h. After completion of the reaction was confirmed by TLC, the reaction liquid under reduced pressure was concentrated to a crude product, and the obtained crude product was purified by column chromatography to obtain a compound 9 (0.02 g, white solid). LC-MS:ESI$[M+H]^+$=427.2. $^1H$ NMR (400 MHz, Chloroform-d) δ 11.47 (s, 1H), 8.48 (d, *J* = 1.8 Hz, 1H), 7.99 (dd, *J* = 7.7, 1.7 Hz, 1H), 7.84-7.72 (m, 2H), 7.64-7.52 (m, 2H), 6.17-6.08 (m, 1H), 3.71 (s, 2H), 3.20 (d, *J* = 3.3 Hz, 2H), 2.95 (d, *J* = 5.0 Hz, 3H), 2.70 (t, *J* = 5.6 Hz, 2H), 2.51 (s, 2H).

**Embodiment 10:**

1'-((7-ethyl-6-oxo-5,6-dihydro-1,5-naphthyridin-3-yl)methyl)-2-fluoro-N-(methyl-$d_3$)-1',2',3', 6'-tetrahydro-[3,4'-bipyridine]-6-carboxamide

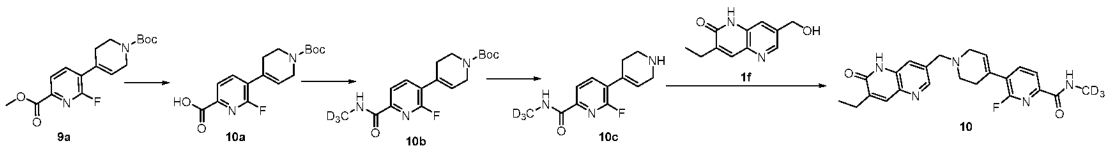

The compound 1'-((7-ethyl-6-oxo-5,6-dihydro-1,5-naphthyridin-3-yl)methyl)-2-fluoro-N -(methyl-$d_3$)-1',2',3',6'-tetrahydro-[3,4'-bipyridine]-6-carboxamide was obtained via a similar procedure as that described for Embodiment 2. LC-MS:ESI$[M+H]^+$=425.2. $^1H$ NMR (400 MHz, Chloroform-d) δ 11.15 (s, 1H), 8.47 (d, *J* = 1.8 Hz, 1H), 7.99 (dd, *J* = 7.7, 1.7 Hz, 1H), 7.83-7.73 (m, 2H), 7.63 (s, 1H), 7.57 (s, 1H), 6.13 (t, *J* = 2.6 Hz, 1H), 3.72 (s, 2H), 3.21 (d, *J* = 3.3 Hz, 2H), 2.75-2.62 (m, 4H), 2.52 (s, 2H), 1.24 (t, *J* = 7.4 Hz, 3H).

**Embodiment 11:**

1'-((7-(ethyl-d5)-6-oxo-5,6-dihydro-1,5-naphthyridin-3-yl)methyl)-N (methyl-$d_3$)-1',2',3',6'-tet rahydro-[3,4'-bipyridine]-6-carboxamide

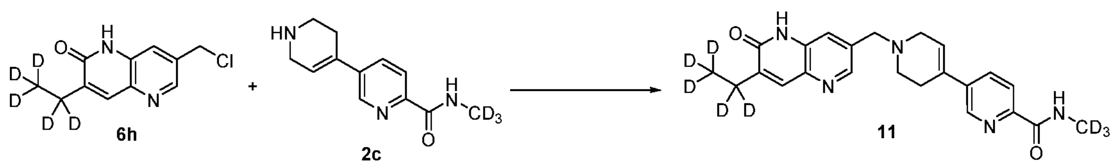

The compound 1'-((7-(ethyl-$d_5$)-6-oxo-5,6-dihydro-1,5-naphthyridin-3-yl)methyl)-N -(methyl-$d_3$)-1',2',3',6'-tetrahydro-[3,4'-bipyridine]-6-carboxamide was obtained via a similar procedure as that described for Embodiment 1. LC-MS:ESI$[M+H]^+$=412.3. $^1H$ NMR (400 MHz, Chloroform-d) δ 11.25 (s, 1H), 8.49 (dd, *J* = 8.7, 2.0 Hz, 2H), 8.08 (d, *J* = 8.2 Hz, 1H), 7.87 (s, 1H), 7.79 (s, 1H), 7.73 (dd, *J* = 8.2, 2.3 Hz, 1H), 7.62 (d, *J* = 1.7 Hz, 1H), 6.15 (t, *J* = 3.7 Hz, 1H), 3.72 (s, 2H), 3.20 (d, *J*= 3.2 Hz, 2H), 2.73 (t, *J* = 5.6 Hz, 2H), 2.54 (s, 2H).

**Embodiment 12:**

*N*-cyclopropyl-1'-((7-(ethyl-$d_5$)-6-oxo-5,6-dihydro-1,5-naphthyridin-3-yl)methyl)-1',2',3',6'-tet rahydro-[3,4'-bipyridine]-6-carboxamide.

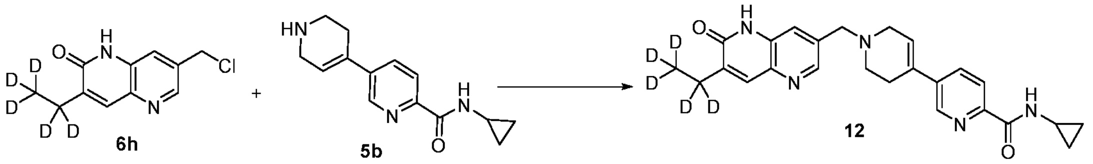


The compound *N*-cyclopropyl-1'-((7-(ethyl-$d_5$)-6-oxo-5,6-dihydro-1,5-naphthyridin-3-yl)methyl)-1',2',3',6'-tetrahydro-[3,4'-bipyridine]-6-carboxamide was obtained via a similar procedure as that described for Embodiment 1. LC-MS:ESI[M+H]$^+$=435.3. $^1$H NMR (400 MHz, Chloroform-*d*) δ 11.53 (s, 1H), 8.55-8.36 (m, 2H), 8.08 (dd, *J* = 8.3, 0.8 Hz, 1H), 7.92 (d, *J* = 3.8 Hz, 1H), 7.80 (s, 1H), 7.72 (dd, *J* = 8.2, 2.3 Hz, 1H), 7.63 (d, *J* = 1.9 Hz, 1H), 6.23-6.06 (m, 1H), 3.72 (s, 2H), 3.20 (d, *J* = 3.1 Hz, 2H), 2.91-2.84 (m, 1H), 2.72 (t, *J* = 5.7 Hz, 2H), 2.53 (s, 2H), 0.86-0.72 (m, 2H), 0.63-0.53 (m, 2H).

**Embodiment 13:**

1'-((2-(ethyl-$d_5$)-5-fluoro-3-oxo-3,4-dihydroquinoxalin-6-yl)methyl)-N-methyl-1',2',3',6'-tetra hydro-[3,4'-bipyridine]-6-carboxamide

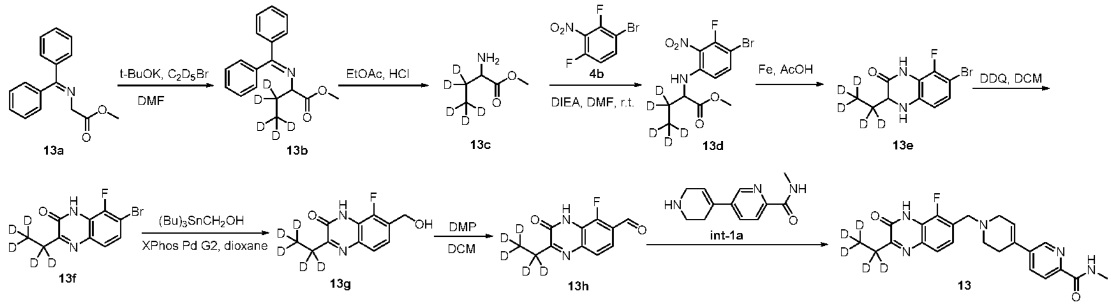


Step 1: The compound 13a (10.0 g, 39.5 mmol) was added into a reaction flask, and dissolved in 100 mL DMF under nitrogen protection. Then, *t*-BuOK (6.6 g, 59.2 mmol) was added into the system in batches at -10°C, and continuously stirred at -10°C for 1 h. After addition, the mixture was slowly dropped in deuteromethyl bromide (5.0 g, 43.4 mmol), and restored to room temperature and stirred for 3 h. 300 mL water was added into the system, and the organic phases were extracted with EA (500 mL × 3), the organic phases were combined and washed with saturated saline solution, the liquid was separated and dried with anhydrous $Na_2SO_4$, filtered, and concentrated in vacuum to obtain a yellow oily liquid 13b (11.0 g, 97%). LC-MS: ESI[M+H]$^+$=287.2.

Step 2: The compound 13b (11.0 g, 38.4 mmol), 120 mL mixed solvent of EA/$H_2O$ (3/1) and 4 M HCL-ethyl acetate solution (28.8 mL, 115.2 mmol) were added. The mixture was stirred and reacted at room temperature overnight, and concentrated in vacuum to remove the solvent, and the slurry was made with EA. The obtained white solid is a hydrochloride (2.5 g, 41%) of 13c. LC-MS: ESI[M+H]$^+$=123.1.

Step 3: The hydrochloride (2.5 g, 15.8 mmol) of 13c was added into a reaction flask, and 30 mL DMF was added to dissolve the hydrochloride of 13c. DIEA (8.1 g, 63.0 mmol) was added into the reaction flask, and stirred at room temperature followed by addition of 4b (4.1 g, 17.3 mmol) into the reaction flask. The mixture was stirred for 5 h at room temperature under nitrogen protection. After the reaction was completed, 90 mL pure water was added, the organic phase was extracted with EA (150 mL × 2), the organic phase was combined and washed with a saturated saline solution, the liquid was separated and concentrated in vacuum, and the organic phase was purified by column chromatography by using PE and DCM (0% - 20%) to obtain yellow oily oil 13d (4.8 g, 90%), LC-MS: ESI[M+H]$^+$=340.0.

Step 4: The compound 13d (4.5 g, 13.2 mmol) was dissolved in 80 mL glacial acetic acid, the system was stirred at room temperature when reducing Fe powder (4.4g, 79.4 mmol) was slowly added. The mixture was stirred for 1 h at 70°C, and filtered before it cools down. The filter cake was washed with a mixed solvent of DCM and MeOH, the filtrate was concentrated in vacuum and purified by column chromatography by using PE and EA (0% - 25%) to obtain white solid 13e (2.1 g, 57%), LC-MS: ESI[M+H]$^+$=278.0.

Step 5: The compound 13e (2.1 g, 7.6 mmol) was added into a reaction flask, 30 mL DCM was added and stirred, then DDQ (2.1 g, 9.1 mmol) was slowly added. The mixture was stirred and reacted at room temperature overnight. After the reaction was completed, a saturated aqueous solution of $NaHCO_3$ was added into the system, the liquid was separated, and the water phase was extracted with a mixed solvent (30 mL × 3) of DCM and MeOH (5:1). The organic phase was combined and dried with anhydrous $Na_2SO_4$, filtered and concentrated, and the dry filtrate was purified by column chromatography by using DCM and MeOH (0% - 3%) to obtain a while solid 13f (2.0 g, 96%), LC-MS: ESI $[M+H]^+$=276.0.

Step 6: The compound 13f (1.2 g, 5.3 mmol), (tributyltin) methanol (2.8 g, 8.7 mmol) and XPhos-Pd-G2 (416.4 mg, 0.4 mmol) were added into a reaction flask, and 30 mL dioxane was added. The nitrogen was replaced for 3-5 times, and the mixture was stirred at 90°C under nitrogen protection for 4 h. After the reaction was completed, the mixture was concentrated under vacuum until the solvent was removed, and the concentrate was purified by column chromatography with DCM and MeOH (0% - 5%) to obtain a white solid, 13g (946.0 mg, 76%), LC-MS: ESI$[M+H]^+$=228.1; $^1H$ NMR (400 MHz, DMSO) δ 12.46 (s, 1H), 7.59 (d, *J* = 8.3 Hz, 1H), 7.37 (t, *J* = 7.7 Hz, 1H), 5.46 (t, *J* = 5.8 Hz, 1H), 4.68 (d, *J* = 5.6 Hz, 2H).

Step 7: The compound 13g (650 mg, 2.9 mmol) was added into a reaction flask, 20 mL DCM was added to dissolve 13g, a Dess-Martin oxidant (1.5 g, 3.4 mmol) was slowly added at 0°C, and the mixture was stirred for 0.5 h at 0°C. After the reaction was completed, a saturated aqueous solution of $NaHCO_3$ was added into the system and stirred for 10 min, the liquid was separated and the organic solvent was concentrated in vacuum until it is dry The concentrate was purified by column chromatography by using DCM and MeOH (0% - 5%) to obtain a while solid 13h (610 mg, 95%), LC-MS: ESI$[M+H]^+$=226.1;$^1H$ NMR (400 MHz, $CDCl_3$) δ 10.37 (s, 1H), 9.85 (s, 1H), 7.72 - 7.63 (m, 2H).

Step 8: The compound int-1a (99.2 mg, 0.34 mmol) was added into a reaction flask, 5 mL DCM was added first and then TEA to free them, followed by dropwise addition of glacial acetic acid until pH is approximately 5. Finally, 13h (70.0 mg, 0.31 mmol) was added and the mixture was stirred at room temperature for 2 h. Triacetoxyborohydride sodium (263.5 mg, 1.24 mmol) was added into the system and stirred for 2 h, followed by addition of a saturated aqueous solution of $NH_4Cl$ and stirring. The liquid was separated and the water phase was extracted with a mixed solvent (30 mL × 3) of DCM and MeOH (5:1), the organic phase was combined and dried with anhydrous $Na_2SO_4$, filtered, and concentrated until the filtrate it is dry, and the residue was processed through pre-HPLC preparation, frozen and dried to obtain a white solid compound **13** (16 mg, 12%). LC-MS: ESI$[M+H]^+$=427.2; $^1H$ NMR (400 MHz, DMSO) δ 12.37 (s, 1H), 8.76 - 8.66 (m, 2H), 8.02 - 7.94 (m, 2H), 7.56 (d, *J* = 8.3 Hz, 1H), 7.32 (t, *J* = 7.6 Hz, 1H), 6.42 (s, 1H), 3.77 (s, 2H), 3.18 (s, 2H), 2.82 (d, *J* = 4.7 Hz, 3H), 2.73 (t, *J* = 5.4 Hz, 2H), 2.55 (s, 2H).

**Embodiment 14:**

1'-((2-(ethyl-$d_5$)-5-fluoro-3-oxo-3,4-dihydroquinoxalin-6-yl)methyl)-2-fluoro-N-methyl-1',2',3 '6'-tetrahydro-[3,4'-bipyridine]-6-carboxamide

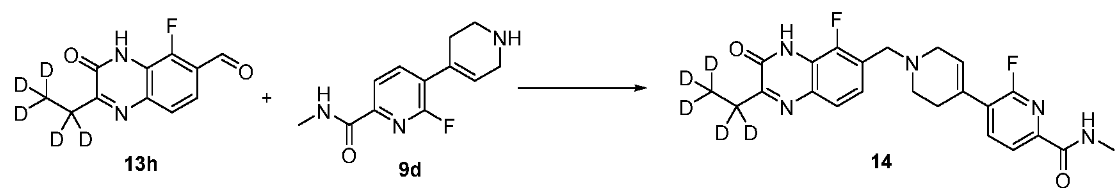

The compound 9d (150.5 mg, 0.49 mmol) was added into a reaction flask, 5 mL DCM was added first and then TEA to free them, followed by dropwise addition of glacial acetic acid until pH was approximately 5. Finally, 13h (100.0 mg, 0.44 mmol) was added and the mixture was stirred at room temperature for 2 h. Triacetoxyborohydride sodium (376.4 mg, 1.78 mmol) was added into the system and stirred for 2 h, followed by addition of a saturated aqueous solution of $NH_4Cl$ and stirring. The liquid was separated and the water phase was extracted with a mixed solvent (30 mL × 3) of DCM and MeOH (5: 1), the organic phase was combined and dried with anhydrous $Na_2SO_4$, filtered, and concentrated until the filtrate it is dry, and the residue was processed through pre-HPLC preparation, frozen and dried to obtain a white solid compound **14** (60 mg, 30%). LC-MS: ESI$[M+H]^+$=445.2; $^1H$ NMR (400 MHz, DMSO) δ 12.47 (s, 1H), 8.72 - 8.58 (m, 1H), 8.08 (dd, *J* = 9.8,7.8 Hz, 1H), 7.92 (dd, *J* = 7.7,1.4 Hz, 1H), 7.56 (d, *J* = 8.3 Hz, 1H), 7.32 (t, *J* = 7.7 Hz, 1H), 6.25 (s, 1H), 3.76 (s, 2H), 3.18 (d, *J* = 2.5 Hz, 2H), 2.80 (d, *J* = 4.8 Hz, 3H), 2.70 (t, *J* = 5.5 Hz, 2H), 2.51 - 2.46 (m, 2H).

**Embodiment 15:**

1'-((7-ethyl-6-oxo-5,6-dihydro-1,5-naphthyridin-3-yl)methyl-$d_2$)-N-methyl-1',2',3',6'-tetrahydr o-[3,4'-bipyridine]-6-carboxamide

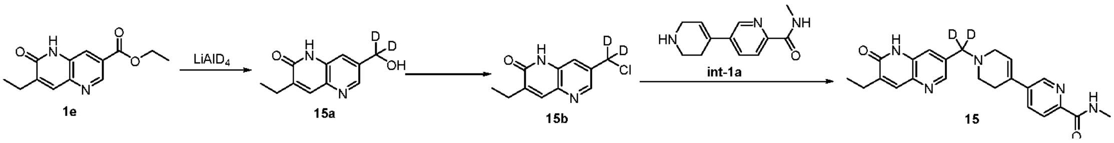


Step 1: In a 150 mL reaction flask, a compound 1e (2.0 g, 8.1 mmol) was added, followed by 60 mL tetrahydrofuran. The mixture was cooled to 0°C, and then a 2.5 mol/L solution of lithium aluminum deuteride was dissolved in tetrahydrofuran (6.48 mL, 16.2 mmol), and reacted at 0°C for 2 h. After completion of the reaction was confirmed by TLC, 5 mL water was added to quench the reaction, followed by a large amount of anhydrous sodium sulfate to dry the mixture. The mixture was filtered, the filter cake was washed with a large amount of dichloromethane, and the filtrate was combined and concentrated by rotary evaporation. The residue was dried to obtain a compound 15a (0.8 g, white solid). LC-MS:ESI[M+H]$^+$=207.3.

Step 2: In a 50 mL reaction flask, a compound 15a (0.82 g, 4.0 mmol) was added, followed by 20 mL dichloromethane and 1 mL *N,N*-dimethylformamide, and the mixture was cooled to 0°C, and thionyl chloride (0.87 mL, 12 mmol) was dropwise added, and reacted at 0°C for 1 h. After completion of the reaction was confirmed by TLC, the reaction liquid was concentrated by rotary evaporation, and the obtained crude product was purified by column chromatography to obtain a compound 15b (0.52g, gray solid). LC-MS:ESI[M+H]$^+$=225.7.

Step 3: The compound 15b (56 mg, 0.25 mmol), INT1 (71 mg, 0.231 mmol), N,N-Diisopropylethylamine (0.17 g, 1.25 mmol) and potassium iodide (0.22 g, 1.25 mmol) were added into 10 mL anhydrous acetonitrile, and stirred at 80°C for 2 h. After completion of the reaction was confirmed by TLC, the reaction liquid under reduced pressure was concentrated to a crude product, and the obtained crude product was purified by column chromatography to obtain a compound **15** (25m g, white solid); LC-MS:ESI[M+H]$^+$=406.4.

## Embodiment 16:

1'-((7-(ethyl-2,2,2-$d_3$)-6-oxo-5,6-dihydro-1,5-naphthyridin-3-yl)methyl)-N-methyl-1',2',3',6'-t etrahydro-[3,4'-bipyridine]-6-carboxamide

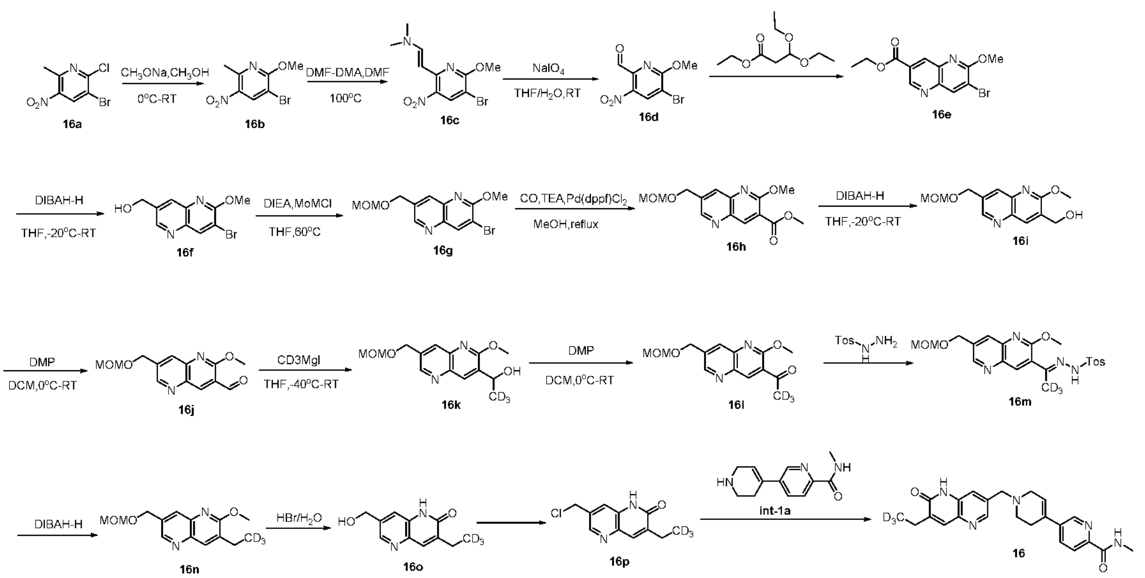


Step 1: The compound 16a (500.0 g, 1.99 mol), and 2.5L methanol were added and stirred and under nitrogen protection, and the mixture was cooled to 0°C - 5°C. Sodium methoxide (118.0g, 2.19 mol), and 1.0 L methanol were added and stirred to dissolve, and then the mixture was dropwise added into a reaction system with the temperature controlled

within 0°C - 5°C. After the addition was completed, the mixture was returned to room temperature naturally, and stirred for 1 h. After completion of the reaction was confirmed by TLC, 2.0 L water was added into the reaction system. Under reduced pressure at 40°C - 50°C, the reaction liquid was concentrated until no liquid is produced. The aqueous layer was extracted twice with ethyl acetate (4.0 L and 1.0 L, respectively), the organic phases were combined and concentrated under reduced pressure at 40°C - 50°C until the weight became unchanged to obtain a compound 16b (480g, white solid).

Step 2: The compound 16b (475 g, 1.93 mol), 2.85 L DMF and 2.85 L DMF-DMA were added and the mixture was heated to 100°C, and stirred for 2 h. After completion of the reaction was confirmed by TLC, the reaction liquid was concentrated at 70°C - 80°C until no more liquid was distilled off. 10.0 L water was added and stirred at 20°C - 30°C for 2 h, and the mixture was filtered. The filter cake was dried under reduced pressure at 60°C - 70°C until its weight became unchanged to obtain a compound 16c (612 g, red solid).

Step 3: The compound 16c (500 g, 1.91 mol), and 2.56 L tetrahydrofuran were added. Sodium periodate (805.0 g, 3.72 mol) was dissolved in 2.56 L water. At a temperature of 25°C - 30°C, the sodium periodate solution was dropwise added into the reaction system, and stirred at 25°C - 30°C for 2 h - 4 h. After completion of the reaction was confirmed by TLC, 2.56 L water and 4.0L ethyl acetate were added and stirred to separate the layers. The aqueous layer was washed twice with 2.0 L ethyl acetate. The organic layers were combined and washed successively with 4.0 L saturated sodium thiosulfate solution and 4.0 L saturated saline solution. The liquid was separated and the organic layer was concentrated under reduced pressure at 40°C - 50°C until its weight became unchanged to obtain a compound 16d (500 g, oily).

Step 4: The compound 16d (500.0 g, 1.69 mol) and 3,3-dideoxypropionic acid ethyl ester (1,457.0 g, 7.61 mol) were added into 7.5 L absolute ethyl alcohol, and stannous chloride (1,815.0 g, 9.57 mol) was added. After addition, the temperature was raised for reflux reaction for 1 h - 2 h. After completion of the reaction was confirmed by TLC, the reaction liquid under reduced pressure was concentrated at 40°C - 50°C until its weight became unchanged. 15.0 L ethyl acetate was added, the pH was adjusted to 7 - 8 with a saturated sodium bicarbonate solution, and the organic layers were filtered and concentrated under reduced pressure at 40°C - 50°C until the weight became unchanged, and purified by column chromatography to obtain a compound 16e (230.0g, white solid).

Step 5: The compound 16e (80.0 g, 257.1 mmol), and 960 mL tetrahydrofuran were added, and the mixture was cooled to 0°C in an ice-water bath. Diisopropylaluminum hydride (1 M, 771.3 mL) was dropwise added, and the temperature was raised to 20°C - 25°C after addition for reaction. After completion of the reaction was confirmed by LC-MS, 800 mL saturated ammonium chloride solution was added into the reaction system, and the filtrate was filtered and concentrated by rotary evaporation. 80 mL dichloromethane was added to pulp, and the filtrate was filtered and concentrated under reduced pressure at 40°C - 50°C until is weight became unchanged to obtain a compound 16f (43.0 g, yellow solid).

Step 6: The compound 16f (45.0 g, 167.2 mmol), DIPEA (64.8 g, 501.6 mmol), sodium iodide (5.0 g, 33.4 mmol), and 450 mL tetrahydrofuran were added. MOMCl (26.9 g, 334.4 mmol) was stirred while being added dropwise. Then, the mixture was heated to 45°C - 50°C and stirred for 3 h. After completion of the reaction was confirmed by TLC, the mixture was cooled to 20°C - 25°C, 500 mL water was added, and the liquid was separated. The aqueous layer was extracted with 300 mL ethyl acetate, and the organic layers were combined. The organic layers were concentrated under reduced pressure at 40°C - 50°C until they were dry, and purified by column chromatography to obtain a compound 16g (34.8 g, yellow solid).

Step 7: The compound 16g (39.8 g, 127.1 mmol), triethylamine (15.5 g, 152.5 mmol) and Pd(dppf)$Cl_2$ (9.22 g, 12.7 mmol) were added, and then 800 mL methanol. The nitrogen was replaced for three times, followed by carbon monoxide three times. The mixture was heated to 60°C and reacted for 12 h. After completion of the reaction was confirmed by LC-MS, the reaction liquid under reduced pressure was concentrated at 40°C - 50°C to remove the solvent, and the residue was purified by column chromatography to obtain a compound 16h (32.0 g, white solid).

Step 8: The compound 16h (32.0 g, 109.5 mmol), and 384 mL tetrahydrofuran were added, and the mixture was cooled to 0°C in an ice-water bath. Diisopropylaluminum (1 M, 328.5 mmol), was dropwise added and then the temperature was returned to 20°C - 25°C after addition for reaction. After completion of the reaction was confirmed by LC-MS, 320 mL saturated ammonium chloride solution and 320 mL ethyl acetate were added into the reaction system, and the filtrate was filtered and concentrated under reduced pressure at 40°C - 50°C to obtain a compound 16i (28.6 g, yellow solid).

Step 9: The compound 16i (28.6 g, 108.2 mmol), and 286 mL dichloromethane were added and stirred for complete dissolving. The mixture was cooled in an ice-water bath to 0°C, and the Dess-Martin periodinane (55.0 g, 129.8 mmol) was added in batches. The temperature was returned to 20°C - 25°C after addition and the reaction was made for 2 h. After completion of the reaction was confirmed by TLC, 300 mL solution of saturated sodium bicarbonate and saturated sodium thiosulfate (1:1) were added and the mixture was washed twice. The aqueous layer was extracted with 300 mL dichloromethane, and the organic layers were combined and concentrated under reduced pressure at 40°C - 50°C, and purified by column chromatography to obtain a compound 16j (23.4 g, yellow solid).

Step 10: The compound 16j (23.4 g, 89.1 mmol), and 234 mL tetrahydrofuran were added, and the mixture was cooled to -40°C in a dry ice bath. Deuterated methyl iodide magnesium (1 M, 106.9 mmol) was dropwise added and the mixture was returned to room temperature naturally, and stirred for 1 h. After completion of the reaction was confirmed by

TLC, 234 mL saturated ammonium chloride solution and 234 mL water were added to quench, stirred to separate the liquid. The organic layers were combined and concentrated under reduced pressure at 40°C - 50°C to obtain a compound 16k (25.6 g, oily).

Step 11: The compound 16k (24.7 g, 88.1 mmol), and 247 mL dichloromethane were added and stirred for complete dissolving. The mixture was cooled in an ice-water bath to 0°C, and the Dess-Martin periodinane (44.8 g, 105.7 mmol) was added in batches. The temperature was returned to 20°C - 25°C after addition and the mixture was reacted for 2 h. After completion of the reaction was confirmed by TLC, 300 mL solution of saturated sodium bicarbonate and saturated sodium thiosulfate (1:1) were added and the mixture was washed twice. The aqueous layer was extracted with 300 mL dichloromethane, and the organic layers were combined and concentrated under reduced pressure at 40°C - 50°C until they are dry, and purified by column chromatography to obtain a compound 16l (23.2 g, yellow solid).

Step 12: The compound 16l (5.0 g, 17.9 mmol), p-toluenesulfonyl hydrazide (3.3 g, 17.9 mmol), and 100 mL methanol were added, and the mixture was reacted at 20°C - 25°C for 16 h. After completion of the reaction was confirmed by TLC, the reaction liquid was filtered, and the filter cake was dried under reduced pressure at 40°C - 50°C until its weight became unchanged to obtain a compound 16m (7.20 g, white solid).

Step 13: The compound 16m (4.50 g, 10.0 mmol), and 45 mL dichloromethane were added, and the mixture was cooled to 0°C in an ice-water bath, and diisopropylaluminum hydride (1 M, 20 mL) was dropwise added. The mixture was heated to 20°C - 25°C for the reaction. After completion of the reaction was confirmed by LC-MS, 20 mL saturated ammonium chloride solution was added into the reaction system, and the filtrate was filtered and concentrated by rotary evaporation, and the obtained product was purified by column chromatography to obtain a compound 16n (540 mg, oily).

Step 14: The compound 16n (400.0 mg, 1.51 mmol), 4 mL dioxane and 4 mL 48% $HBr/H_2O$ were added, and the mixture was heated to 80°C, and the mixture was reacted for 2 h. After completion of the reaction was confirmed by LC-MS, the pH to 7 - 8 was adjusted by using the saturated sodium carbonate solution, and the filter cake was filtered and purified by column chromatography to obtain a compound 16o (200 mg, white solid).

Step 15: The compound 16o (100.0 mg, 0.48 mmol), 3 mL toluene and DMF (3.5 mg, 0.048 mmol) were added, and the mixture was cooled to 0°C in an ice-water bath. Thionyl chloride (69.0 mg, 0.48 mmol) was dropwise added, then the mixture was heated to 20°C - 25°C, and the mixture was reacted. After completion of the reaction was confirmed by TLC-MS, the mixture was concentrated under reduced pressure to remove the solvent and obtain a compound 16p (108 mg, yellow solid).

Step 16: The compound 16p (108.0 mg, 0.48 mmol), int-1 (139.0 mg, 0.48 mmol), DIEA (248.0 mg, 1.92 mmol), and KI (16.0 mg, 0.096 mmol) were added into 3 mL acetonitrile. The mixture was heated to 80°C and reacted for 2 h. After completion of the reaction was confirmed by LC-MS, the mixture was cooled to room temperature and concentrated by rotary evaporation, and the obtained crude product was purified by column chromatography to obtain a compound **16** (100 mg, white solid); LC-MS:ESI$[M+H]^+$=407.2; 1H NMR (400 MHz, DMSO-d6) δ 11.89 (s, 1H), 8.82 - 8.71 (m, 2H), 8.48 (d, *J* = 1.8 Hz, 1H), 8.10 - 7.98 (m, 2H), 7.81 (s, 1H), 7.70 (d, *J* = 1.8 Hz, 1H), 6.48 (d, *J* = 3.6 Hz, 1H), 3.78 (s, 2H), 3.22 (q, *J* = 3.0 Hz, 2H), 2.88 (d, *J* = 4.8 Hz, 3H), 2.77 (t, *J* = 5.6 Hz, 2H), 2.62 (d, *J* = 6.2 Hz, 2H), 2.58 (s, 2H).

**Embodiment 17:**

1'-((7-(ethyl-2,2,2-$d_3$)-6-oxo-5,6-dihydro-1,5-naphthyridin-3-yl)methyl)-1',2',3',6'-tetrahydro-[ 2,4'-bipyridine]-5-carbonitrile

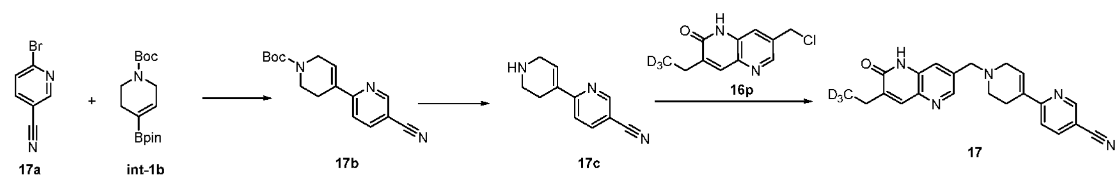

Step 1: The compound 17a (0.5 g, 2.7 mmol), int-1b (1.02 g, 3.3 mmol), $Pd(dppf)Cl_2$ (0.2 g, 0.27 mmol) and potassium carbonate (0.94 g, 6.8 mmol) were added into a mixed solvent of 7 mL dioxane, 3 mL absolute ethyl alcohol and 4 mL water, then nitrogen was replaced for three times, and the mixture was reacted at 90°C under nitrogen protection for 2 h. After completion of the reaction was confirmed by TLC, the reaction mixture was cooled to room temperature, 30 mL dichloromethane and 20 mL water were added, and the layers were separated in a separatory funnel. The water phase was extracted twice with dichloromethane, the organic phases were combined and then washed sequentially with water and saturated brine. The reaction liquid was dried with anhydrous sodium sulfate, filtered, and concentrated by rotary evaporation. The obtained crude product was purified by column chromatography to obtain a compound 17b (0.7 g, white

solid).

Step 2: The compound 17b (0.5 g, 1.8 mmol) was added into 10 mL absolute methanol, followed by 10 mL solution of hydrochloric acid and dioxane (4 mol/L), and stirred at room temperature for 0.5 - 1 h. After completion of the reaction was confirmed by TLC, the reaction liquid under reduced pressure was concentrated to a dry compound 17c (0.2 g, white solid).

Step 3: The compound 17c (0.05 g, 0.27 mmol), 16p (0.072 g, 0.32 mmol), N,N-diisopropylethylamine (0.17 g, 1.35 mmol) and potassium iodide (0.22 g, 1.35 mmol) were added into 10 mL anhydrous acetonitrile, and stirred at 80°C for 2 h. After completion of the reaction was confirmed by TLC, the reaction liquid under reduced pressure was concentrated to a crude product, and the obtained crude product was purified by column chromatography to obtain a compound 17 (0.021 g, white solid). LC-MS:ESI[M+H]$^+$=375.2. $^1$H NMR (400 MHz, DMSO-$d_6$) δ 11.85 (s, 1H), 8.96 (dd, *J* = 2.2, 0.8 Hz, 1H), 8.42 (d, *J* = 1.8 Hz, 1H), 8.26 (dd, *J* = 8.4, 2.3 Hz, 1H), 7.75 (d, *J* = 8.3 Hz, 2H), 7.65 (d, *J* = 1.9 Hz, 1H), 6.95 (dd, *J* = 4.5, 2.6 Hz, 1H), 3.74 (s, 2H), 3.22 (d, *J* = 3.4 Hz, 2H), 2.70 (t, *J* = 5.6 Hz, 2H), 2.60 (s, 2H).

**Embodiment 18:**

3-(ethyl-2,2,2-$d_3$)-7-((5-fluoro-3',6'-dihydro-[2,4'-bipyridin]-1'(2'H)-yl)methyl)-1,5-naphthyri din-2(1H)-one

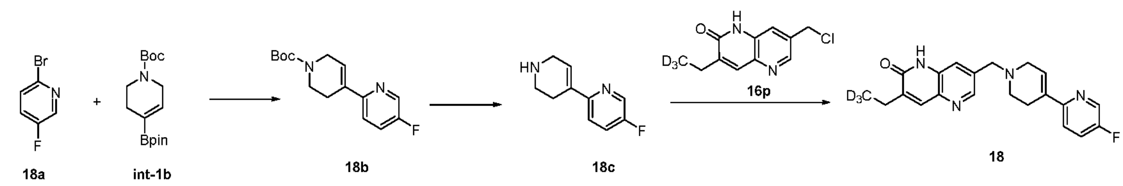

18a int-1b 18b 18c 16p 18

The compound 3-(ethyl-2,2,2-$d_3$)-7-((5-fluoro-3',6'-dihydro-[2,4'-bipyridin]-1'(2'H)-yl)methyl)-1,5-naphthyridin-2(1H)-one was obtained via a similar procedure as that described for Embodiment 17. LC-MS:ESI[M+H]$^+$=368.2. $^1$H NMR (400 MHz, DMSO-$d_6$) δ 11.85 (s, 1H), 8.52 (d, *J* = 2.9 Hz, 1H), 8.42 (d, *J* = 1.9 Hz, 1H), 7.76 (s, 1H), 7.70 (td, *J* = 8.8, 3.0 Hz, 1H), 7.67-7.60 (m, 2H), 6.64 (s, 1H), 3.73 (s, 2H), 3.16 (d, *J* = 3.5 Hz, 2H), 2.69 (t, *J* = 5.6 Hz, 2H), 2.58 (s, 2H).

**Embodiment 19:**

3-(ethyl-$d_5$)-8-fluoro-7-((5-fluoro-3',6'-dihydro-[2,4'-bipyridin]-1'(2'H)-yl)methyl)quinoxalin-2(1H)-one

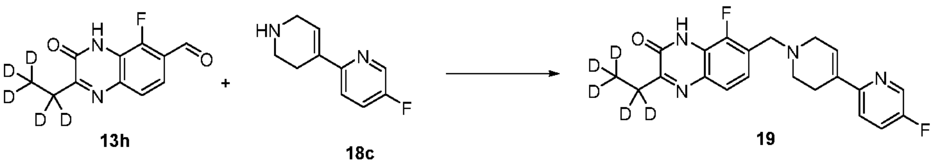

13h 18c 19

The compound 18c (122.6 mg, 0.49 mmol) was added into a reaction flask, 5 mL DCM was added and then TEA was added to free them, followed by dropwise addition of glacial acetic acid until pH was approximately 5. Finally, 13h (100.0 mg, 0.44 mmol) was added and the mixture was stirred at room temperature for 2 h. Triacetoxyborohydride sodium (376.4 mg, 1.78 mmol) was added into the system and stirred for 2 h, and a saturated aqueous solution of $NH_4Cl$ was added and stirred. The liquid was separated, and the water phase was extracted with a mixed solvent (30 mL × 3) of DCM and MeOH (5:1). The organic phase was combined and dried with anhydrous $Na_2SO_4$, filtered, and concentrated until the filtrate was dry. The residue was processed through pre-HPLC preparation, frozen and dried to obtain a white solid compound 19 (32 mg, 19%). LC-MS: ESI[M+H]$^+$=388.2; $^1$H NMR (400 MHz, DMSO) δ 12.42 (s, 1H), 8.49 (d, *J* = 2.8 Hz, 1H), 7.67 (td, *J* = 8.7, 2.9 Hz, 1H), 7.60 (dd, *J* = 8.9, 4.5 Hz, 1H), 7.54 (d, *J* = 8.3 Hz, 1H), 7.34 - 7.27 (m, 1H), 6.61 (s, 1H), 3.75 (s, 2H), 3.16 (d, *J* = 2.3 Hz, 2H), 2.69 (t, *J* = 5.5 Hz, 2H), 2.55 (s, 2H).

**Embodiment 20:**

1'-((2-(ethyl-$d_5$)-5-fluoro-3-oxo-3,4-dihydroquinoxalin-6-yl)methyl)-1',2',3',6'-tetrahydro-[2,4' -bipyridine]-5-carbonitrile

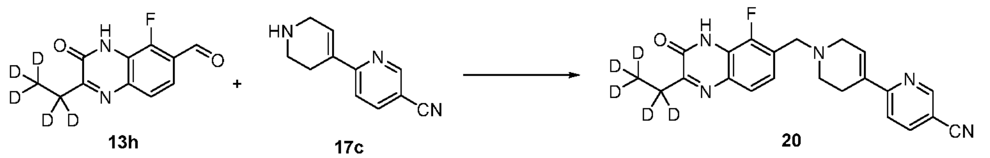

The compound 17c (126.1 mg, 0.49 mmol) was added into a reaction flask, 5 mL DCM was added first and then TEA was added to free them, followed by dropwise addition of glacial acetic acid until pH was approximately 5. Finally, 13h (100.0 mg, 0.44 mmol) was added and the mixture was stirred at room temperature for 2 h. Triacetoxyborohydride sodium (376.4 mg, 1.78 mmol) was added into the system, continue stirring for 2 h, a saturated aqueous solution of $NH_4Cl$ was added and stirred. The liquid was separated, and the water phase was extracted with a mixed solvent (30 mL × 3) of DCM and MeOH (5:1). The organic phase was combined and dried with anhydrous $Na_2SO_4$, filtered, and concentrated until the filtrate was dry. The residue was processed through pre-HPLC preparation, frozen and dried to obtain a white solid compound **20** (14 mg, 8%). LC-MS: ESI[M+H]$^+$=395.20; $^1$H NMR (400 MHz, DMSO) δ 8.94 (s, 1H), 8.28 - 8.19 (m, 1H), 7.72 (d, *J* = 8.4 Hz, 1H), 7.55 (d, *J* = 8.2 Hz, 1H), 7.31 (t, *J* = 7.6 Hz, 1H), 6.92 (s, 1H), 3.77 (s, 2H), 3.22 (s, 2H), 2.70 (d, *J* = 5.2 Hz, 2H), 2.57 (s, 2H).

**Embodiment 21:**

1'-((7-ethyl-6-oxo-5,6-dihydro-1,5-naphthyridin-3-yl)methyl)-N-methyl-1',2',3',6'-tetrahydro-[ 3,4'-bipyridine]-2',2',6',6'-$d_4$-6-carboxamide

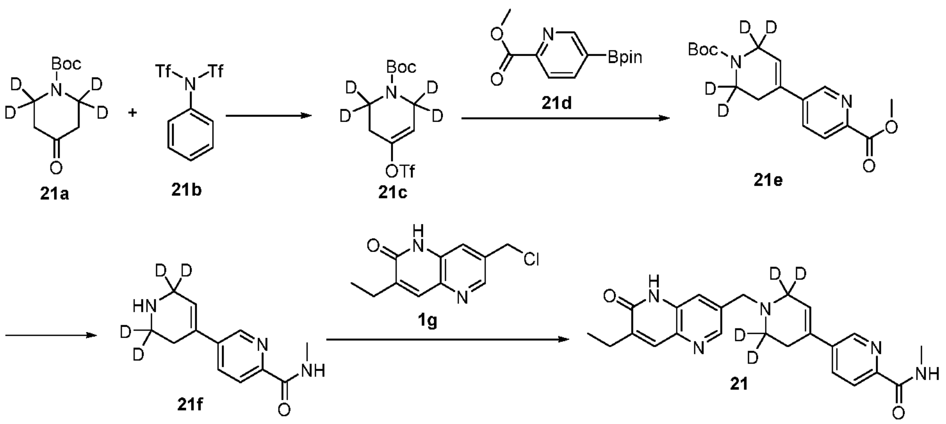

Step 1: The compound 21a (6.0 g, 49.2 mmol) was added into 50 mL tetrahydrofuran and the mixture was cooled to -78°C. While the temperature was maintained between -60°C and -78°C, LDA (2 M, 29.5 mL, 59.0 mmol) was slowly dropwise added. After the addition was completed, the mixture was stirred for 15 min, then a 40 mL tetrahydrofuran solution of 21b (19.3 g, 54.1 mmol) was dropwise added. The reaction liquid was allowed to be warmed naturally to room temperature and stirred for 2 h. After completion of the reaction was confirmed by TLC, 60 mL aqueous solution of saturated ammonium chloride was added and stirred to separate the liquid. Then the aqueous layer was extracted with 100 mL ethyl acetate and the liquid was separated again. The organic phases were combined and washed with 100 mL saturated saline solution. After phase separation was completed, the organic layer was concentrated under reduced pressure and the solvent was removed to obtain a compound 21c (20.0 g, oily).

Step 2: The compound 21c (19.3 g, 28.7 mmol), potassium carbonate (7.93 g, 57.4 mmol), and 21d (8.32 g, 31.6 mmol) were added into 100 mL solution of 1,4-dioxane/water (10:1). The nitrogen was replaced for three times, and then the mixture was heated to 80°C, and stirred for 2 h. After completion of the reaction was confirmed by LC-MS, the mixture was cooled to room temperature. 100 mL water and 100 mL ethyl acetate were added, and the layers were separated. The aqueous layer was extracted with 100 mL ethyl acetate three times. The organic layers were combined and dried with anhydrous sodium sulfate, and the filtrate was filtered and concentrated by rotary evaporation, and the obtained crude

product was purified by column chromatography to obtain a compound 21e (4.2 g, yellow solid).

Step 3: The compound 21e (4.2 g, 13.0 mmol) was added into 21 mL absolute methanol, then 40% aqueous solution of methylamine (5.1 g, 65.2 mmol) was added and stirred at room temperature for 1 h. After completion of the reaction was confirmed by LC-MS, the reaction liquid was concentrated until it was dry. 40 mL 1,4-dioxane was added, and then 4 M/hydrochloric acid and dioxane solution (16.3 mL, 65.2 mmol) were dropwise added. The mixture was stirred at room temperature for 2 h. After completion of the reaction was confirmed by LC-MS, the filtrate was filtered and concentrated by rotary evaporation to obtain a compound 21f (3.4 g, off-white solid).

Step 4: The compound 21f (3.4 g, 11.5 mmol), DIEA (5.6 g, 46.0 mmol), a compound 1g (2.4 g, 10.5 mmol), and KI (340 mg, 2.1 mmol) were added into 48 ml of acetonitrile. The mixture was heated to 80°C and stirred for 2 hours. After completion of the reaction was confirmed by LC-MS, 200 mL saturated sodium bicarbonate solution was added and stirred, and the filtrate was filtered and concentrated by rotary evaporation, and purified by column chromatography to obtain a compound **21** (2.4 g, white solid), LC-MS:ESI[M+H]$^+$=408.2; $^1$H NMR (400 MHz, DMSO-$d_6$) δ 11.90 (s, 1H), 8.82-8.72 (m, 2H), 8.48 (d, *J* = 1.9 Hz, 1H), 8.10-7.99 (m, 2H), 7.82 (q, *J* = 1.0 Hz, 1H), 7.75-7.66 (m, 1H), 6.48 (d, *J* = 1.5 Hz, 1H), 3.78 (s, 2H), 2.88 (d, *J* = 4.9 Hz, 3H), 2.64-2.58 (m, 4H), 1.25 (t, *J* = 7.4 Hz, 3H).

**Embodiment 22:**

1'-((7-ethyl-6-oxo-5,6-dihydro-1,5-naphthyridin-3-yl)methyl)-2-fluoro-N-methyl-1',2',3',6'-tet rahydro-[3,4'-bipyridine]-2',2',6',6'-$d_4$-6-carboxamide

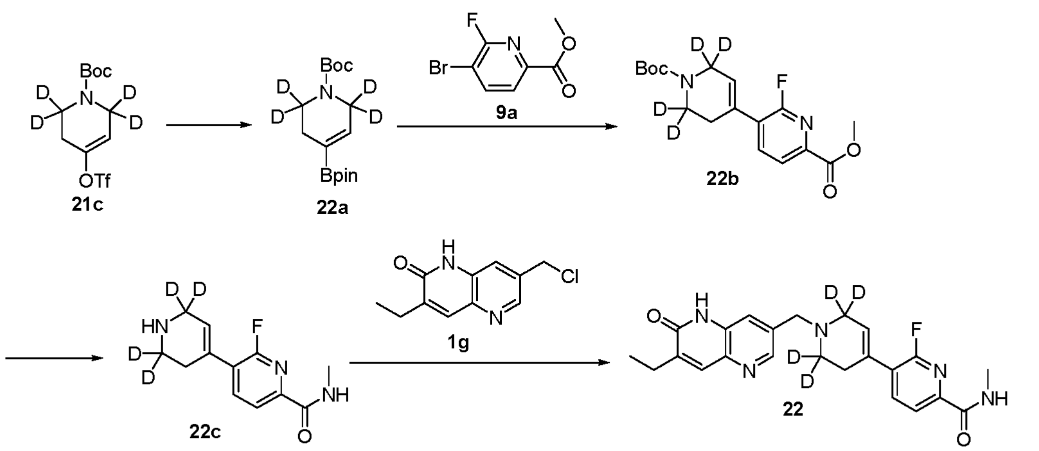

Step 1: The compound 21c (20 g, 60 mmol), pinacol diboronate (15.2 g, 72 mmol), potassium acetate (11.8 g, 120 mmol), and PdCl$_2$(dppf) (2.1 g, 3 mmol) were added sequentially into a reaction flask, and reacted under $N_2$ protection at 80°C overnight. After the reaction was completed, the reaction liquid was filtered and washed with EA, the filtrate was concentrated and the residue was purified by column chromatography to obtain a compound 22a (15 g, oily).

Step 2: The compound 22a (15 g, 22.3 mmol), potassium carbonate (7.93 g, 57.4 mmol), and 9a (8.32 g, 31.6 mmol) were added into 100 mL solution of 1,4-dioxane/water (10:1). The nitrogen was replaced for three times, and then the mixture was heated to 80°C, and stirred for 2 h. After completion of the reaction was confirmed by LC-MS, the mixture was cooled to room temperature. 100 mL water and 100 mL ethyl acetate were added, and the layers were separated. The aqueous layer was extracted with 100 mL ethyl acetate three times. The organic layers were combined and dried with anhydrous sodium sulfate, filtered and concentrated by rotary evaporation, and the obtained crude product was purified by column chromatography to obtain a compound 22b (12 g, yellow solid).

Step 3: The compound 22b (4.2 g, 13.0 mmol) was added into 21 mL absolute methanol, then 40% aqueous solution of methylamine (5.1 g, 65.2 mmol) was added and stirred at room temperature for 1 h. After completion of the reaction was confirmed by LC-MS, the reaction liquid was concentrated until it was dry. 40 mL 1,4-dioxane was added, and then 4 M/hydrochloric acid and dioxane solution (16.3 mL, 65.2 mmol) were dropwise added and stirred at room temperature for 2 h. After completion of the reaction was confirmed by LC-MS, the filtrate was filtered and concentrated by rotary evaporation to obtain a compound 22c (3.4 g, off-white solid).

Step 4: The compound 22c (3.4 g, 11.5 mmol), DIEA(5.6 g, 46.0 mmol), the compound 1g (2.4 g, 10.5 mmol), and KI (340 mg, 2.1 mmol) were added into 48 mL acetonitrile. The mixture was heated to 80°C and stirred for 2 h. After completion of the reaction was confirmed by LC-MS, 200 mL saturated sodium bicarbonate solution was added and stirred, and the filtrate was filtered and concentrated by rotary evaporation and purified by column chromatography to

obtain a compound **22** (2.4 g, white solid); LC-MS:ESI[M+H]$^+$=426.5; $^1$H NMR (400 MHz, DMSO) δ 11.84 (s, 1H), 8.63 (d, *J* = 4.8 Hz, 1H), 8.42 (t, *J* = 4.4 Hz, 1H), 8.16 - 8.03 (m, 1H), 7.92 (dd, *J* = 7.7, 1.5 Hz, 1H), 7.75 (s, 1H), 7.65 (s, 1H), 6.24 (s, 1H), 3.72 (s, 2H), 2.88 (t, *J* = 8.1 Hz, 1H), 2.80 (d, *J* = 4.8 Hz, 3H), 2.60 - 2.52 (m, 3H), 1.18 (t, *J* = 7.4 Hz, 3H).

**Embodiment 23:**

1'-((7-ethyl-6-oxo-5,6-dihydro-1,5-naphthyridin-3-yl)methyl)-N,2-dimethyl-1',2',3',6'-tetrahyd ro-[3,4'-bipyridine]-2',2',6',6'-$d_4$-6-carboxamide

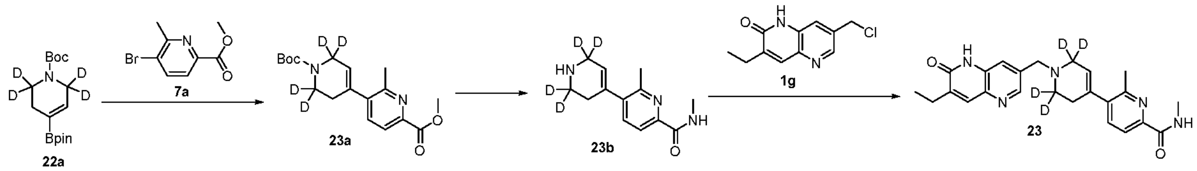

Step 1: The compound 22a (13 g, 21.2 mmol), potassium carbonate (7.8 g, 55 mmol), and 100 mL 7a (8.1 g, 28.6 mmol) were added into 100 mL solution of 1,4-dioxane/water (10:1). The nitrogen was replaced for three times, then the mixture was heated to 80°C, and stirred for 2 h. After completion of the reaction was confirmed by LC-MS, the mixture was cooled to room temperature. 100 mL water and 100 mL ethyl acetate were added to separate the layers. The aqueous layer was extracted with 100 mL ethyl acetate three times. The organic layers were combined and dried with anhydrous sodium sulfate, fand the filtrate was filtered and concentrated by rotary evaporation, and the obtained crude product was purified by column chromatography to obtain a compound 23a (10.6 g, yellow solid).

Step 2: The compound 23a (3.8 g, 11.3 mmol) was added into 21 mL absolute methanol, then 40% aqueous solution of methylamine (4.5 g, 60.5 mmol) was added and stirred at room temperature for 1 h. After completion of the reaction was confirmed by LC-MS, the reaction liquid was concentrated until it was dry. 40 mL 1,4-dioxane was added, and then 4 M/hydrochloric acid and dioxane solution (15 mL, 64 mmol) was dropwise added and stirred at room temperature for 2 h. After completion of the reaction was confirmed by LC-MS, the filtrate was filtered and concentrated by rotary evaporation to obtain a compound 23b (3.2 g, off-white solid).

Step 3: The compound 23b (3.2 g, 11.2 mmol), DIEA(5.4 g, 45.2 mmol), the compound 1g (2.1 g, 10.5 mmol), and KI (320 mg, 1.9 mmol) were added into 48 mL acetonitrile. The mixture was heated to 80°C and stirred for 2 h. After completion of the reaction was confirmed by LC-MS, 200 mL saturated sodium bicarbonate solution was added and stirred, and the filtrate was filtered and concentrated by rotary evaporation and purified by column chromatography to obtain a compound **23** (1.8 g, white solid); LC-MS:ESI[M+H]$^+$=426.5; $^1$H NMR (400 MHz, DMSO) δ 11.84 (s, 1H), 8.63 (d, *J* = 4.8 Hz, 1H), 8.42 (t, *J* = 4.4 Hz, 1H), 8.16 - 8.03 (m, 1H), 7.92 (dd, *J* = 7.7, 1.5 Hz, 1H), 7.75 (s, 1H), 7.65 (s, 1H), 6.24 (s, 1H), 3.72 (s, 2H), 2.88 (t, *J* = 8.1 Hz, 1H), 2.80 (d, *J* = 4.8 Hz, 3H), 2.60 - 2.52 (m, 3H), 1.18 (t, *J* = 7.4 Hz, 3H).

**Biological activity test:**

**1. Determination of PARP-1/2/5a enzyme**

Experimental materials: PARP1 protein (BPS, Cat. No. 80501), PARP2 protein (BPS, Cat. No. 80502), PARP5A protein (BPS, Cat. No. 80504), Biotin-NAD+ (R&D, Cat. No. 6573), Strep-HRP (Thermo Pierce, Cat. No. 21127), NAD+ (TCI, Cat. No. D0919-5G), QuantaRed Enhanced HRP Substrate Kit (Thermo Pierce, Cat. No. 15159), Histone (Active Motif, Cat. No. 81167), Activated DNA (Genscript, Cat. No. L05182-01&02&03), anti-rabbit IgG, HRP-linked Antibody (CST, Cat. No. 7074P2), anti-Poly/Mono-ADP Ribose (E6F6A) Rabbit mAb (CST, Cat. No. 83732S), SuperSignal ELISA Femto Substrate (THERMO PIERCE, Cat. No. 37074), and the reference compound AZD5305 bought from MedChem-Express (MCE).

**1.1 Determination of PARP1 enzyme**

1.1.1 Preparation of buffer solution: PBST: 1x PBS, 0.05% Tween-20; blocking solution: 1x PBS, 0.05% Tween-20, 5% BSA; reaction buffer: 50 mM Tris-HCl (pH 7.5), 0.005% Tween-20, 0.01% BSA.
1.1.2 Coating: A 50 ng/mL Histone coating solution was prepared by using 1x PBS, 25 uL of the coating solution was

transferred to a 384-well reaction plate, and the reaction plate was coated overnight at 4°C.
1.1.3 Washing: After the coating was completed, the coating solution was discarded, and the reaction plate was washed with the PBST solution by means of transferring 50 uL of the PBST to the 384-well reaction plate, letting it stand for 5 min, discarding the washing solution, refilling the reaction plate , and repeating the washing process 3 times. Finally, the reaction plate was tapped dry and the next step of blocking was proceeded with.
1.1.4 Blocking: 50 uL of the blocking solution was transferred to the 384-well reaction plate and set for 1 h.
Washing: After the blocking was completed, the blocking solution was discarded, the reaction plate was washed 3 times with the PBST solution according to the method in Step 2, and the reaction plate was tapped dry.
1.1.5 A 1,000x compound was prepared, 1 μL of the compound was transferred to a 96-well plate containing 199 μL of the reaction buffer, mixed well, and then 5 μL of the mixed compound was transferred to the 384-well reaction plate.
1.1.6 A 25/10x PARP1-DNA solution was prepared by using the reaction buffer, 10 uL of the PARP1-DNA solution was transferred to the 384-well reaction plate. For a negative control well, 10 uL of the DNA solution was transferred and the final concentration of PARP1 was 0.02 nM and that of DNA was 0.8 nM.
1.1.7 A 25/10x NAD+ solution was prepared by using the reaction buffer, and 10 uL of the NAD+ solution was transferred to the 384-well plate, resulting in the final NAD+ concentration of 3.5 uM, and the reaction plate was incubated at room temperature for 60 min.
1.1.8 A 25/10x NAD+ solution was prepared by using the reaction buffer, and 10 uL of the NAD+ solution was transferred to the 384-well plate, resulting in the final NAD+ concentration of 3.5 uM, and the reaction plate was incubated at room temperature for 60 min.
1.1.9 Washing: After the reaction was completed, the reaction liquid was discarded. The reaction plate was washed 3 times with the PBST solution according to the method in Step 2, and the reaction plate was tapped dry.
1.1.10 A 2,000x anti-Poly/Mono-ADP Ribose Rabbit mAb was diluted with the blocking solution, 20 uL of the diluted anti-Poly/Mono-ADP Ribose Rabbit was added, and incubated at room temperature for 1.5 h.
1.1.11 Washing: The anti-Poly/Mono-ADP Ribose Rabbit was discarded, the reaction plate was washed 3 times with the PBST solution according to the method in Step 2, and the reaction plate was tapped dry.
1.1.12 A 2,000x anti-rabbit IgG, HRP-linked Antibody was diluted with the blocking solution, 20 uL of the diluted anti-rabbit IgG, HRP-linked Antibody was added, and incubated at room temperature for 1 h.
1.1.13 Washing: The anti-rabbit IgG, HRP-linked Antibody was discarded, the reaction plate was washed 3 times with the PBST solution according to the method in Step 2, and the reaction plate was tapped dry.
1.1.14 Color developing: Femto-ECL Substrate A and Femto-ECL Substrate B were mixed in a 1:1 ratio, and 25 uL of the mixture was transferred to the 384-well reaction plate.
1.1.15 Reading: A relative light unit (RLU) was read with Envision.

**1.2 Determination of PARP2 enzyme**

1.2.1 Preparation of buffer solution: PBST: 1x PBS, 0.05% Tween-20; blocking solution: 1x PBS, 0.05% Tween-20, 5% BSA; reaction buffer solution: 50 mM HEPES (pH 7.5), 0.002% Tween-20, 0.1% BSA, 100 mM NaCl, 2mM DTT.
1.2.2 Coating: A 100 ng/mL Histone coating solution was prepared by using 1x PBS, 25 uL of the coating solution was transferred to a 384-well reaction plate, and the reaction plate was coated overnight at 4°C.
1.2.3 Washing: After the coating was completed, the coating solution was discarded, and the reaction plate was washed with the PBST solution by means of transferring 50uL of the PBST solution to the 384-well reaction plate, letting it stand for 5 min, discarding the washing solution, refilling the reaction plate, and repeating the washing process 3 times. Finally, the reaction plate was tapped dry and the next step of blocking was proceeded with.
1.2.4 Blocking: 50 uL of the blocking solution was transferred to the 384-well reaction plate and set for 1 h.
1.2.5 Washing: After the blocking was completed, the blocking solution was discarded, the reaction plate was washed 3 times with the PBST solution according to the method in Step 2, and the reaction plate was tapped dry.
1.2.6 A 25/10× PARP2 solution was prepared, then 10 μL of the PARP2 solution was transferred to the 384-well reaction plate. For negative control wells, 10 μL of the reaction buffer solution instead was transferred, resulting in the final concentration of PARP2 of 1.5 nM.
1.2.7 A 2,000x compound was prepared, 50 nL of the compound with echo was transferred, 19.95 uL of the reaction buffer solution was added into the compound, mixed well, and 5 uL of the mixed compound was transferred to the 384-well reaction plate.
1.2.8 A 25/10x Biotin-NAD+ solution was prepared, 10uL of the Biotin-NAD+ solution was transferred to the 384-well reaction plate, resulting in a final Biotin-NAD+ concentration of 2uM, and the reaction plate was incubated at room temperature for 60 min.
1.2.9 Washing: After the reaction was completed, the reaction liquid was discarded. The reaction plate was washed 3

times with the PBST solution according to the method in Step 2, and the reaction plate was tapped dry.

1.2.10 A Stre-HRP solution was prepared by means of diluting it with the blocking solution, 25 uL of the Stre-HRP solution was transferred to the reaction plate, and incubated at room temperature for 1 h, resulting the final concentration of Stre-HRP of 0.1 μg/mL.

1.2.11 Washing: Discard the Stre-HRP solution, wash the reaction plate 3 times with the PBST solution according to the method in Step 2, and the reaction plate was tapped dry.

1.2.12 Color developing: Femto-ECL Substrate A, Femto-ECL Substrate B and QuantaRed ADHP were mixed in a ratio of 50:50:1. 25 uL of the mixture was transferred to the 384-well reaction plate and incubated at room temperature for 10 min and 2.5 uL QuantaRed Stop Solution was added.

1.2.13 Reading: A fluorescence value (Ex550/Em 620) was read with Paradigm.

**1.3 Determination of PARP5A enzyme**

1.3.1 Preparation of buffer solution solution: PBST: 1x PBS, 0.05% Tween-20; blocking solution: 1x PBS, 0.05% Tween-20, 5% BSA; reaction buffer solution: 50 mM HEPES (pH 7.5), 0.002% Tween-20, 0.1% BSA, 100 mM NaCl, 2mM DTT.

1.3.2 Coating: A 100 ng/mL Histone coating solution was prepared by using 1x PBS, 25 uL of the coating solution was transferred to a 384-well reaction plate, and the reaction plate was coated overnight at 4°C.

1.3.3 Washing: After the coating was completed, the coating solution was discarded, and the reaction plate was washed with the PBST solution by means of transferring 50uL of the PBST solution to the 384-well reaction plate, letting it stand for 5 min, discarding the washing solution, refilling the reaction plate with the PBST solution, and repeating the washing process 3 times. Finally, the reaction plate was tapped dry and the next step of blocking was proceeded with.

1.3.4 Blocking: 50 uL of the blocking solution was transferred to the 384-well reaction plate and set for 1 h.

1.3.5 Washing: After the blocking was completed, the blocking solution was discarded, the reaction plate was washed 3 times with the PBST solution according to the method in Step 2, and the reaction plate was tapped dry.

1.3.6 A 25/10× PARP5A solution was prepared, then 10 μL of the PARP5A solution was transferred to the 384-well reaction plate. For negative control wells, 10 μL of the reaction buffer solution instead was transferred, resulting in the final concentration of PARP5A of 10 nM.

1.3.7 A 2,000x compound was prepared, 50 nL of the compound was transferred to with echo, 19.95 uL of the reaction buffer solution was added into the compound, mix well, and 5 uL of the mixed compound was transferred to the 384-well reaction plate.

1.3.8 A 25/10x Biotin-NAD+ solution was prepared, 10uL of the Biotin-NAD+ solution was transferred to the 384-well reaction plate, resulting in a final Biotin-NAD+ concentration of 2uM, and incubated at room temperature for 60 min.

1.3.9 Washing: After the reaction was completed, the reaction liquid was discarded. The reaction plate was washed 3 times with the PBST solution according to the method in Step 2, and the reaction plate was tapped dry.

1.3.10 A Stre-HRP solution was prepared by means of diluting it with the blocking solution, 25uL of the Stre-HRP solution was transferred to the reaction plate, and incubated at room temperature for 1 h, resulting the final concentration of Stre-HRP of 0.1 μg/mL.

1.3.11 Washing: The Stre-HRP solution was discarded, the reaction plate was washed 3 times with the PBST solution according to the method in Step 2, and the reaction plate was tapped dry.

1.3.12 Color developing: Femto-ECL Substrate A, Femto-ECL Substrate B, and QuantaRed ADHP were mixed in a 50:50:1 ratio. 25 μL of the mixture was transferred to the 384-well reaction plate, incubated at room temperature for 10 min, and 2.5 μL of QuantaRed Stop Solution was added.

1.3.13 Reading: Afluorescence value (Ex550/Em 620) was read with Paradigm.

Test results are shown in Table 2:

Table 2 Test results of PARP-1/2/5a enzyme

| Compound | PARP-1 inhibition ratio | | PARP-2 | PARP-5a |
|---|---|---|---|---|
| | 10 nM | 100 nM | $IC_{50}$ (nM) | $IC_{50}$ (nM) |
| **1** | 99.6% | 99.6% | > 1000 | > 1000 |
| **6** | 99.6% | 99.7% | > 1000 | > 1000 |
| **7** | 99.8% | 99.8% | > 1000 | > 1000 |

(continued)

| Compound | PARP-1 inhibition ratio | | PARP-2 | PARP-5a |
|---|---|---|---|---|
| | 10 nM | 100 nM | $IC_{50}$ (nM) | $IC_{50}$ (nM) |
| **9** | 99.7% | 99.7% | > 1000 | > 1000 |
| **11** | 99.6% | 99.6% | > 1000 | > 1000 |
| **12** | 99.8% | 99.9% | > 1000 | > 1000 |
| **13** | 96.1% | 98.5% | > 1000 | > 1000 |
| **14** | 98.9% | 99.1% | > 1000 | > 1000 |
| **16** | 99.5% | 99.6% | > 1000 | > 1000 |
| **AZD5305** | 99.5% | 99.6% | - | - |

Conclusion: The compounds of the invention exhibit significant inhibitory effects on PARP1, while showing weak inhibition on PARP2/5a, indicating that the compounds of the invention selectively inhibit PARP-1.

**2. Anti-proliferative activity assay of cell:**

BRCA-mutated MDA-MB-436 cells were cultured in a DMEM medium, which contain 10% fetal bovine serum, 100 U/mL penicillin and 100 μg/mL streptomycin, and the cells were incubated at 37°C in a 5% saturated $CO_2$ incubator. When reached an 80% confluence, the cells are collected and centrifuged at 300 g for 10 min, and then spread in 96-well plates at a density of 1,200 cells per well. After 24 h, PARPi varying in final concentration (0, 0.01, 0.1, 1, 10, 100, and 1000 nM) was added and the cells were further cultured for 72 h. The cells were subjected to fluid exchange treatment (with the same final concentration of PARPi added again), and then cultured for 96 h. The 96-well plate was taken out and the CCK8 method was adopted to measure the OD value at a wavelength of 450 nm. Then, the cell inhibition rate was calculated by using the following formula: Inhibition rate (%) = 1 - (average OD value of the treatment group - Average OD value of the Blank group) / (average OD value of the Control group - Average OD value of the Blank group) * 100%.

BRCA wild-type DLD-1 cells were cultured in a RPMI-1640 medium, which contain 10% fetal bovine serum, 100 U/mL penicillin and 100 μg/mL streptomycin, and the cells were incubated at 37°C in a 5% saturated $CO_2$ incubator. When reached an 80% confluence, the cells are collected and centrifuged at 300 g for 10 min, and spread in 96-well plates at a density of 1,000 cells per well. After 24 h, PARPi varying in final concentration (0, 1, and 10 μM) was added and the cells were further cultured for 72 h. The cells were subjected to fluid exchange treatment (with the same final concentration of PARPi added again), and then cultured for 96 h. The 96-well plate was taken out and the CCK8 method was adopted to measure the OD value at a wavelength of 450 nm, and the cell inhibition rate was calculated:

Inhibition rate (%) = 1 - (average OD value of the treatment group - Average OD value of the Blank group) / (average OD value of the Control group - Average OD value of the Blank group) * 100%.

Test results are shown in Table 3 below:

Table 3 Test results of anti-proliferation activity of cells

| Compound | MDA-MB-436 $IC_{50}$ (nM) | DLD-1 $IC_{50}$ (nM) | Compound | MDA-MB-436 $IC_{50}$ (nM) | DLD-1 $IC_{50}$ (nM) |
|---|---|---|---|---|---|
| **1** | < 1 (0.9) | >10000 | **2** | 1.1 | >10000 |
| **3** | 1 | >10000 | **4** | 6 | >10000 |
| **5** | 0.7 | >10000 | **6** | 0.6 | >10000 |
| **7** | 1.5 | >10000 | **8** | 0.8 | >10000 |
| **9** | 1.1 | >10000 | **10** | 0.6 | >10000 |
| **11** | 0.8 | >10000 | **12** | 0.9 | >10000 |
| **13** | 8.2 | >10000 | **14** | 0.95 | >10000 |
| **16** | 0.7 | >10000 | **17** | 13.5 | >10000 |

(continued)

| Compound | MDA-MB-436 $IC_{50}$ (nM) | DLD-1 $IC_{50}$ (nM) | Compound | MDA-MB-436 $IC_{50}$ (nM) | DLD-1 $IC_{50}$ (nM) |
|---|---|---|---|---|---|
| **18** | 39.2 | >10000 | **21** | 1 | >10000 |
| **22** | 0.7 | >10000 | **23** | 0.6 | >10000 |
| **AZD5305** | 1 | - | | | |

Conclusion: The compounds of the invention exhibit significant inhibitory effects on BRCA-mutated MDA-MB-436 cells, while showing no obvious inhibitory effects on BRCA wild-type DLD-1 cells, indicating that the compounds of the invention specifically inhibit tumor cells with homologous recombination deficiencies.

**3. Pharmacokinetic evaluation of compound in Balb/c mice**

Experimental purpose: To learn pharmacokinetic profiles of compounds.
Experimental basis: Technical Guidelines for Non-clinical Pharmacokinetic Studies of Chemical Drugs, 2014.
Experimental scheme: The pharmacokinetic profile of the compound was investigated by means of administering it intravenously (1 mg·$kg^{-1}$) and orally (1 mg·$kg^{-1}$) to Balb/c mice.
Sample preparation: About 0.2 mg of the compound was dissolved in 10 μL DMSO, then a sodium chloride injection was added to make a 0.1 mg·$mL^{-1}$ solution of the compound for administration.
Sample collection: Six male Balb/c mice (Chengdu Dossy Experimental Animal Co., Ltd., License No.: SCXK (C) 2020-030) were used. Three mice were administered the compound intravenously (IV) at a dose of 1 mg·$kg^{-1}$, and the other three were administered the compound orally (PO) at the same dose. Approximately 0.05 mL blood samples were collected at 5 min, 15 min, 30 min, 1 h, 2 h, 4 h, 6 h, 8 h, 10 h, 24 h, and 48 h after the administration, respectively. The collected blood samples were centrifuged at 3,500 rpm for 15 min to collect the supernatant plasma, which was then stored at -40°C until assay. The plasma concentration of the drug was quantitatively analyzed with the LC-MS/MS method, and pharmacokinetic parameters were calculated, including the time to peak concentration (Cmax), area under the concentration-time curve (AUC(0-t)), half-life ($T_{1/2}$), clearance (CL), volume of distribution at steady state (Vdss), and bioavailability (F).
The pharmacokinetic evaluation results are presented in Table 4 below:

Table 4 Pharmacokinetic test results of compound in Balb/c mice

| Compound | Dosage Route of administration | Cmax ug/L | AUC(0-t) ug/L*h | $T_{1/2}$ h | CL L/h/kg | Vdss L/kg | F % |
|---|---|---|---|---|---|---|---|
| **AZD5305** | 1 mg/kg, PO | 6299 | 125698 | 14.69 | 0.01 | 0.16 | - |
| **1** | 1 mg/kg, PO | 7607 | 234568 | 19.68 | 0.01 | 0.11 | - |
| **2** | 1 mg/kg, PO | 7622 | 141251 | 14.27 | 0.01 | 0.14 | - |
| **3** | 1 mg/kg, PO | 7464 | 122078 | 11.51 | 0.01 | 0.14 | - |
| **4** | 1 mg/kg, PO | 7374 | 122078 | 11.51 | 0.01 | 0.14 | - |
| **6** | 1 mg/kg, PO | 8208 | 187955 | 16.25 | 0.01 | 0.12 | - |
| **7** | 1 mg/kg, PO | 5992 | 39496 | 4.08 | 0.03 | 0.16 | - |
| **8** | 1 mg/kg, PO | 5007 | 42314 | 4.10 | 0.02 | 0.14 | - |
| **9** | 1 mg/kg, PO | 2503 | 26767 | 6.21 | 0.03 | 0.26 | - |
| **10** | 1 mg/kg, PO | 3842 | 44587 | 4.64 | 0.02 | 0.15 | - |
| **11** | 1 mg/kg, PO | 6586 | 90955 | 15.57 | 0.01 | 0.16 | - |
| **12** | 1 mg/kg, PO | 2294 | 6947 | 3.87 | 0.15 | 0.70 | - |
| **15** | 1 mg/kg, PO | 6415 | 115370 | 14.07 | 0.01 | 0.03 | - |
| **16** | 1 mg/kg, PO | 7162 | 88147 | 8.92 | 0.01 | 0.12 | - |
| **21** | 1 mg/kg, PO | 7428 | 101828 | 9.85 | 0.01 | 0.13 | - |

(continued)

| Compound | Dosage Route of administration | Cmax ug/L | AUC(0-t) ug/L*h | $T_{1/2}$ h | CL L/h/kg | Vdss L/kg | F % |
|---|---|---|---|---|---|---|---|
| **23** | 1 mg/kg, PO | 5118 | 52376 | 8.3 | 0.02 | 0.23 | - |

Conclusion: The compounds of the invention exhibit favorable pharmacokinetic properties in Balb/c mice, including good oral bioavailability, exposure levels, half-life, and clearance rate. When administered orally at a dose of 1 mg/kg, the $C_{max}$ of the compound **6** was superior to that of the reference compound **5305,** as well as compounds **1, 2, 3, 15,** and **21.**

**4. Pharmacokinetic evaluation of compound in SD rat**

Experimental purpose: To learn pharmacokinetic profiles of compounds.
Experimental basis: Technical Guidelines for Non-clinical Pharmacokinetic Studies of Chemical Drugs, 2014.
Experimental scheme: The pharmacokinetic profile of the compound was investigated by oral intravenous administration in SD rats.
Experimental procedure: The compound, a small amount of DMSO and a sodium chloride injection were added to prepare the final solution for administration. The compound was orally administered to 6 male SD rats. Approximately 0.1 mL blood samples were collected at 5 min, 15 min, 30 min, 1 h, 2 h, 4 h, 6 h, 8 h, 10 h, and 24 h after administration, respectively. The blood samples were centrifuged at 3,500 rpm for 15 min, and the supernatant plasma was collected. 5 μL of the plasma was transferred into an EP tube, 100 μL acetonitrile precipitate proteins containing 20 $ng \cdot mL^{-1}$ internal standard SAHA was added, vortexed for 30 s, and centrifuged at 13,000 rpm for 15 min. The supernatant was collected and transferred into a sample vial for assay. Range of standard curve: 10 - 10,000 $ng \cdot mL^{-1}$.
The pharmacokinetic evaluation results are presented in Table 5 below:

Table 5 Pharmacokinetic test results of compound in SD rat

| Compound | Dosage Route of administration | $C_{max}$ ug/L | AUC(0-t) ug/L*h | $T_{1/2}$ h | CL L/h/kg | $V_{dss}$ L/kg | F % |
|---|---|---|---|---|---|---|---|
| **AZD5305** | 2.5 mg/kg, PO | 5096 | 77267 | 10.85 | 0.03 | 0.35 | - |
| **1** | 2.5 mg/kg, PO | 5745 | 95685 | 14.37 | 0.02 | 0.37 | - |
| **6** | 2.5 mg/kg, PO | 6400 | 90384 | 8.40 | 0.03 | 0.33 | - |
| **21** | 2.5 mg/kg, PO | 5437 | 78514 | 9.80 | 0.03 | 0.36 | - |

Conclusion: The compounds of the invention exhibit favorable pharmacokinetic properties in SD rats, including good oral bioavailability, exposure levels, half-life, and clearance rate. $C_{max}$ of the compound 6 was superior to those of the reference compound **AZD5305,** compound **1** and compound **21.**

**5. In-vivo pharmacodynamic study of compounds 1 and 6 on subcutaneous xenograft tumor model of breast cancer MDA-MB-436 in nude mice**

(1) Main instrument and equipment

$CO_2$ cell culture incubator: Yamato, IP610; Biosafety cabinet: Suzhou Antai, BSC-1304IIA2; Benchtop centrifuge: Thermo Scientific, SORVALL ST 16.
Digital inverted microscope: Olympus, CKX3-SLP; constant temperature water bath: Shanghai Yuejin Medical Equipment Co., Ltd., HSW-420; cell counting chamber: Shanghai Qiujing Biochemical Reagent Instrument Co., Ltd., XB.K.25; liquid nitrogen tank: Thermo, CY50935-70; refrigerator: Haier, BCD-601WDGX; medical low-temperature refrigerator: Thermo, ULTS1651.
Water purifier: Millipore, F7PNO9748; Vertical pressure sterilizer: Yamato, DKN812C; 1 mL disposable sterilized syringe: Shanghai Kindly Enterprise Development Group Co., Ltd.; 1 mL disposable sterile insulin syringe: BD; Scale: Shanghai Hengping Instrument Co., Ltd., JY2002; Analytical balance: SARTORIUS, BCE95I-1CEU.
Hygrothermograph: Wuqiang Hygrothermograph Manufacturing Center, LZJZ30260102; Small centrifuge: Thermo, SORVALL LEGEND MICRO 17 Centrifuge; Mini mixer: Yeasen, ES-VM25; Metal bath: SCILOGEX, SCL120-S.

(2) Main software and data processing system

Graphpad Prism, version 6.0, from Graphpad Software, Inc., was used for organizing, quantifying data, and creating bar charts.

(3) Experimental animal

Strain: NOD/SCID; Grade: SPF; Source: Beijing Vitron River Bio-technology Co., Ltd.

(4) Molding method

Culture and preparation of tumor cell: Conventional tumor cell lines were passaged and cultured with a culture medium containing 10% fetal bovine serum, 100 U/mL penicillin, and 100 μg/mL streptomycin. When reached an 80% confluence, the cells were collected and centrifuged at 300 g for 10 min. The cells were washed 3 times with pre-cooled PBS, centrifuged at 300 g for 10 min, collected, re-suspended in PBS, and counted with a blood cell counting chamber, and the cell concentration was adjusted. After cell collection, the cell suspension was pre-cooled on ice. 100 μL of the cell suspension was administered via subcutaneous injection into the dorsal flank of the mice, and the grouped treatment was started when the tumor volume reached 200-300 $mm^3$.

(5) Observation and detection indicator

General observation: Observation of animals: all live experimental animals to be observed; Observation time: twice a day; Observation items: including but not limited to general performance, behavioral status, eyes, mouth, nose and mouth, ears, hair, stool, urine, genitalia and other toxic symptoms; If abnormalities are observed, provide a detailed description.

(6) Weight

Animal assay: all viable experimental animals scheduled for assay; Assay time: All animals were weighed before the modeling for test grouping. After modeling, the mice were weighed once every 2 days, and the weight change of the mice was recorded in real time. Effective criteria: tumor volume (TV), relative tumor volume (RTV) and relative tumor growth rate of subcutaneously implanted tumor model. The long diameter and short diameter of the tumor was measured with vernier calipers every 2 days, and the anti-tumor effects of the tested drug was dynamically observed. The calculation formula of tumor volume (TV) was: TV ($mm^3$) = a×b2×0.5, where a and b were the long diameter and short diameter, respectively.

Based on the measurement results, the relative tumor volume (RTV) was calculated as per the formula: RTV = Vt / V0, where V0 is the tumor volume (TV) measured for each group at the start of treatment (i.e., d0), and Vt is the tumor volume measured at each subsequent time point for that group. The evaluation index for anti-tumor activity was the relative tumor growth rate T/C (%), and the calculation formula was as follows:

$$T/C(\%) = TRTV/CRTV \times 100\%;$$

Where, TRTV is relative tumor volume of the treatment group; CRTV is relative tumor volume of the negative control group. Therapeutic evaluation criteria: T/C (%)> 60 is considered ineffective; T/C (%) ≤ 60, and P < 0.05 (through statistical analysis) is considered effective. Measurement of tumor weight and tumor inhibition rate (%): At the end of the treatment period, the animals were euthanize, tumor masses dissected and isolated, tumors weighed, and photographs took. The tumor inhibition rate (%) was calculated by the formula below:

Tumor inhibition rate (tumor growth inhibition rate, %) = [(average tumor weight (g) of negative control group - average tumor weight (g) of treatment group)] / average tumor weight (g) of negative control group × 100%;

Effective evaluation criteria: Tumor inhibition rate (i.e., tumor growth inhibition rate) < 40% was considered ineffective; tumor inhibition rate ≥ 40% and P < 0.05 (through statistical analysis) was considered effective. Comprehensive evaluation criteria for subcutaneous tumor model: Among the two aforementioned effective evaluation criteria (relative tumor proliferation rate and tumor inhibition rate), if one meets the effective standard, it is judged as effective.

(7) Data acquisition and analysis

All raw data within the facility were collected manually according to the experimental scheme and the relevant guidelines of the research institution, or gathered by using a data acquisition system. Manually collected data can be transcribed into software such as Excel for analysis and reporting. The data from each group were quantitative, and the experimental data for each group of animals in the tables were expressed as mean ± standard deviation (Mean ± SD). In the figures, the experimental data ware described as mean ± standard error of the mean (Mean ± SEM). Statistical analysis, including t-tests and survival analysis, was performed by using the software such as EXCEL and GraphPad Prism.

Experimental procedure: A total of 42 female NOD/SCID mice were used in the experiment and randomly divided into seven groups: a blank control group, three compound **6** dose groups (0.03, 0.1, and 0.3 mg/kg), a reference compound group (AZD5305 0.1 mg/kg), a compound 1 group (0.1 mg/kg), and an Olaparib group (100 mg/kg). Each group consisted of 6 mice, all of which are administered orally once daily. The body weight of the mice was measured every 2 days, and the length and width of the tumors with a vernier caliper. After 24 days of administration, the tumor-bearing mice were anesthetized and euthanized. The tumor tissues were dissected, weighed, and photographed, and the tumor inhibition rate was calculated.

Conclusion: The experimental results are shown in Fig. 1 and Table 6. After cell inoculation, each treatment group was administered continuously for 24 days. Compared to the blank control group, all treatment groups **(AZD5305** group: 0.1 mg/kg, compound **1** group: 0.1 mg/kg, Olaparib group: 100 mg/kg, and compound 6 groups: 0.03, 0.1, and 0.3 mg/kg) showed significant inhibitory effects on tumor growth in the NOD/SCID mice subcutaneous tumor model of human breast cancer cell line MM436. The tumor inhibition rates for each treatment group are 80.79%, 82.18%, 62.19%, 49.77%, 86.73%, and 95.83%, respectively. The relative tumor proliferation rates (T/C%) are 15.2%, 17.0%, 33.4%, 40.8%, 12.6%, and 3.8%, respectively. Among all treatment groups, the compound 6 group (0.3 mg/kg) exhibited the best tumor inhibition effect, with complete tumor regression observed in 2 out of 6 mice (2/6) at the end of treatment. The tumor inhibition effect of the compound 6 group (0.1 mg/kg) was superior to that of the reference compound **AZD5305** and the compound **1** at the same dose, and significantly better than that of the reference drug Olaparib group (100 mg/kg) (P < 0.01). Additionally, there were significant dose-dependent differences among the low, medium, and high dose groups of compound **6** (P < 0.001, P < 0.01).

Table 6 Tumor inhibition rates of compounds 1 and 6 in MDA-MB-436 nude mice model over 24 days

| Group | Dosage (mg/kg) | Qty. | Body weight (g) X±SD | | Tumor volume ($mm^3$) | | | RTV | Tumor weight (g) | |
|---|---|---|---|---|---|---|---|---|---|---|
| | | Start/end | | | X±SD | | T/C% | | | |
| | | | Start | End | Start | End | | | X±SD | Tumor inhibition rate (%) |
| **Blank group** | - | 6/6 | 22.0±0.9 | 22.7±1.0 | 271.9±54.6 | 1791.9±780.0 | - | 6.6 | 2.16±0.38 | - |
| **Olaparib** | 100 | 6/6 | 21.9±1.1 | 21.7±1.4 | 250.7±70.0 | 551.5±343.1 | 33.4 | 2.2 | 0.82±0.39 | 62.19 |
| **AZD5305** | 0.1 | 6/6 | 21.9±0.7 | 21.8±1.0 | 270.5±57.5 | 270.3±87.4 | 15.2 | 1.0 | 0.42±0.17 | 80.79 |
| **Compound 1** | 0.1 | 6/6 | 21.6±0.3 | 21.6±1.1 | 271.4±76.6 | 304.9±141.7 | 17.0 | 1.1 | 0.39±0.28 | 82.18 |
| **Compound 6** | 0.03 | 6/6 | 22.6±0.7 | 21.6±0.7 | 275.0±68.1 | 739.4±308.7 | 40.8 | 2.7 | 1.09±0.29 | 49.77 |
| | 0.1 | 6/6 | 22.3±1.0 | 21.6±0.7 | 274.7±73.8 | 228.0±75.9 | 12.6 | 0.8 | 0.29±0.09 | 86.73 |
| | 0.3 | 6/6 | 22.3±0.8 | 22.0±0.9 | 281.9±67.4 | 71.0±62.5 | 3.8 | 0.3 | 0.09±0.08 | 95.83 |

vs blank group: c, P<0.001

**6. Challenge experiment of compounds 1, 6, and 21 on subcutaneous Xenograft tumor model of breast cancer MDA-MB-436 in nude mice**

Experimental procedure: 15 female NOD/SCID mice were used in the experiment, which were randomly divided into three groups, the compound **1** (1 mg/kg) dose group, the compound **6** (1 mg/kg) dose group, and the compound **21** (1 mg/kg) dose group. Each group consisted of 5 mice and was administered orally once per day. The body weight of the mice was measured every 2 days, and the length and width of the tumors with a vernier caliper.

Conclusion: Experimental results are shown in Fig. 2. The compounds **1, 6** and **21** of the invention still exhibit excellent anti-tumor efficacy in the model of large-volume tumors and can lead to complete regression of tumors. The compound **6** exhibits a faster rate of tumor regression compared to the compounds **1** and **21,** indicating that, at the same dose, the compound **6** has stronger in-vivo anti-tumor efficacy than compounds **1** and **21.**

**7. In-vivo pharmacodynamic study of compound 6 on PDX gastric cancer mice model**

Experimental procedure: Tumor tissues from gastric cancer patients were used to establish a stable passaged tumor-bearing mice model. In this study, tumor fragments from mice that had been stably passaged three times were used for subcutaneous inoculation. Eight groups were divided for the experiment, with 6 mice in each group. The negative control group (Control) received normal saline at 0.2 mL per mice per administration, given orally once per day; the carboplatin control group (Carboplatin, Car) received 20 mg/kg at 0.2 mL per mice per administration, administered via intraperitoneal injection once every 7 days. Test compound 6: 0.3, 1, and 3 mg/kg, 0.2 mL per mice per administration, given orally once daily; the combination group received the compound 6 + carboplatin.

The details of dosing regimen and grouping are shown in Table 7.

Table 7 Experimental grouping

| Group type (6 mice per group) | Dose (mg/kg) | Route of administration | Administration frequency |
|---|---|---|---|
| Control | - | p.o | Once per day |
| Carboplatin | 20 | i.p | Once per 7 days |
| Compound 6 | 0.3 | p.o | Once per day |
| | 1 | p.o | Once per day |
| | 3 | p.o | Once per day |
| Compound 6+Car | 0.3+20 | p.o+i.p | Car is administered once every 7 days, while the compound **6** is administered once daily. |
| | 1+20 | p.o+i.p | |
| | 3+20 | p.o+i.p | |

Conclusion: Experimental results are shown in Fig. 3. Different doses of compound 6, when combined with carboplatin, exhibit a dose-dependent inhibitory effect on the gastric cancer PDX model. Particularly, the 3 mg/kg dose group of the compound 6 combined with carboplatin significantly inhibit tumor growth.

**8. In-vivo pharmacodynamic study of compound 6 on subcutaneous xenograft tumor model of breast cancer SUM14PT in nude mice**

Experimental procedure: In this study, a mice model was established by subcutaneously inoculating SUM14PT tumor cells. Eight groups were divided for the experiment, with 6 mice in each group. The negative control group (Control) received normal saline at 0.2 mL per mice per administration, given orally once per day; the carboplatin control group (Carboplatin, Car) received 20 mg/kg at 0.2 mL per mice per administration, administered via intraperitoneal injection once every 7 days. Olaparib control group: 100 mg/kg, 0.2 mL per mice per administration, given orally once daily; test compound **6** groups (0.3 and 1 mg/kg): 0.2 mL per mice per administration, given orally once daily; the combination groups received either Olaparib or the compound **6** + carboplatin.

The details of dosing regimen and grouping are shown in Table 8.

Table 8 Experimental grouping

| Group type (6 mice per group) | Dose (mg/kg) | Route of administration | Administration frequency |
|---|---|---|---|
| Control | - | p.o | Once per day |
| Carboplatin | 20 | i.p | Once per 7 days |
| Olaparib | 100 | p.o | Once per day |
| Compound 6 | 0.3 | p.o | Once per day |
| | 1 | p.o | Once per day |
| Olaparib+Car | 100+20 | p.o+i.p | Car: once every 7 days,<br>Olaparib: once per day |
| Compound **6**+Car | 0.3+20 | p.o+i.p | Car is administered once every 7 days, while the compound **6** is administered once daily. |
| | 1+20 | p.o+i.p | |

Conclusion: Experimental results are shown in Fig. 4. The compound **6,** at doses of 0.3 and 1 mg/kg, was administered orally once daily as a monotherapy, showing better anti-tumor efficacy compared to Olaparib at 100 mg/kg administered orally once daily as a monotherapy. Additionally, the compound **6,** administered orally at a dose of 1 mg/kg once daily in combination with carboplatin demonstrated better therapeutic efficacy than Olaparib administered orally at 100 mg/kg once daily in combination with carboplatin.

## Claims

1. The compound represented by Formula II or a pharmaceutically acceptable form thereof, **characterized in that** the Formula II has the following structure:

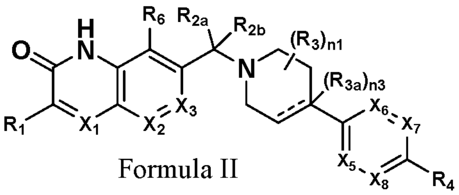

Formula II

Where,

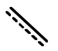 Represents a single bond or a double bond;
$R_1$ is selected from $C_{1-4}$ deuterated alkyl;
$X_1$ is selected from N or $C(R_{5a})$, $X_2$ is selected from N or $C(R_{5b})$, and $X_3$ is selected from N or $C(R_{5c})$, with exactly one of $X_1$, $X_2$, and $X_3$ selected from N;
$R_{2a}$ and $R_{2b}$ are independently selected from hydrogen, deuterium, methyl, or deuteromethyl;
$R_3$ is selected from at least one of deuterium, fluorine, $C_{1-4}$ alkyl, or $C_{1-4}$ deuterated alkyl;
$R_{3a}$ is selected from hydrogen, deuterium, fluorine, hydroxyl, cyano, $C_{1-4}$ alkyl, $C_{1-4}$ fluoroalkyl, or $C_{1-4}$ alkoxy;
$R_4$ is selected from hydrogen, halogen, cyano, $C_{1-4}$ alkyl, $C_{1-4}$ deuterated alkyl, $C_{1-4}$ fluoroalkyl, or $-CONHR_7$;
$R_{5a}$, $R_{5a}$ and $R_{5c}$ are independently selected from hydrogen, fluorine, chlorine, $C_{1-4}$ alkyl, $C_{1-4}$ deuterated alkyl, $C_{1-4}$ fluoroalkyl, $C_{1-4}$ alkoxy or $C_{1-4}$ fluoroalkoxy;
$R_6$ is selected from hydrogen, fluorine, chlorine, $C_{1-4}$ alkyl, $C_{1-4}$ fluoroalkyl, or $C_{1-4}$ deuterated alkyl;
$R_7$ is selected from hydrogen, $C_{1-4}$ alkyl, $C_{1-4}$ deuterated alkyl, $C_{1-4}$ fluoroalkyl, or a 3-6 membered cycloalkyl;
$X_5$ is selected from nitrogen or $C(R_{9a})$, $X_6$ is selected from nitrogen or $C(R_{9b})$, $X_7$ is selected from nitrogen or $C(R_{9c})$, and $X_8$ is selected from nitrogen or $C(R_{9d})$;
$R_{9a}$, $R_{9b}$, $R_{9c}$, and $R_{9d}$ are independently selected from hydrogen, fluorine, chlorine, cyano, $C_{1-4}$ alkyl, $C_{1-4}$ deuterated alkyl, $C_{1-4}$ fluoroalkyl, $C_{1-4}$ alkoxy, or $C_{1-4}$ fluoroalkoxy;
n1 is an integer selected from 0 to 8;
n3 is independently selected from 0 or 1;

The pharmaceutically acceptable form is selected from the group consisting of a pharmaceutically acceptable salt, ester, stereoisomer, polymorph, solvate, N-oxide, isotopically labeled compound, metabolite, or prodrug.

**2.** The compound according to claim 1, **characterized in that** $R_1$ is selected from deuterated methyl or deuteromethyl.

**3.** The compound according to claim 2, **characterized in that** $R_1$ is selected from $-CD_2CD_3$ or $-CH_2CD_3$.

**4.** The compound according to any one of claims 1 to 3, **characterized in that** $R_3$ is selected from at least one of deuterium, methyl, deuterated methyl, fluoromethyl, or methoxy group.

**5.** The compound according to claim 4, **characterized in that** $R_3$ is selected from deuterium or methyl.

**6.** The compound according to any one of claims 1 to 5, **characterized in that** when N is a double bond, $R_{3a}$ does not exist; and when 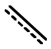 is a single bond, $R_{3a}$ is selected from hydrogen and deuterium.

**7.** The compound according to any one of claims 1 to 6, **characterized in that** $R_4$ is selected from fluorine, chlorine, cyano, $C_{1-4}$ alkyl, $C_{1-4}$ deuterated alkyl, $C_{1-4}$ fluoroalkyl, $C_{1-4}$ alkylaminocarbonyl, $C_{1-4}$ deuterated alkylaminocarbonyl, $C_{1-4}$ fluoroalkylaminocarbonyl, or 3-6 membered cycloalkylaminocarbonyl;
Preferably, $R_4$ is selected from fluorine, cyano, methylaminocarbonyl, deuteriomethylaminocarbonyl, or cyclopropylaminocarbonyl.

**8.** The compound according to any one of claims 1 to 7, **characterized in that** $R_6$ is selected from hydrogen, fluorine, chlorine, or methyl.

**9.** The compound according to any one of claims 1 to 8, **characterized in that** $R_{5a}$, $R_{5b}$, and $R_{5c}$ are independently selected from hydrogen, fluorine, chlorine, or methyl.

**10.** The compound according to any one of claims 1 to 9, **characterized in that** it has a structure represented by Formula VI-1:

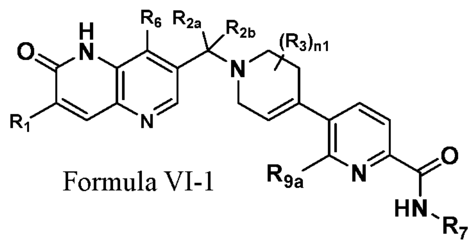

Formula VI-1

Where,

$R_6$ is selected from hydrogen or fluorine;
$R_{9a}$ is selected from hydrogen, fluorine, chlorine, cyano, $C_{1-4}$ alkyl, $C_{1-4}$ deuterated alkyl, or $C_{1-4}$ fluoroalkyl;
n1 is an integer selected from 0 to 6.

**11.** The compound or a pharmaceutically acceptable form thereof according to any one of claims 1 to 10, **characterized in that** it has a structure of Formula VI-3:

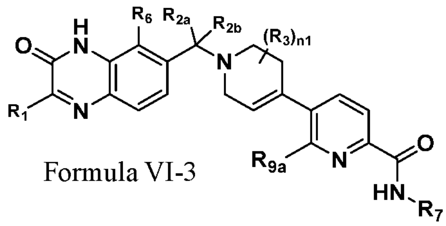

Formula VI-3

Where,

$R_6$ is selected from hydrogen or fluorine;
$R_{9a}$ is selected from hydrogen, fluorine, chlorine, cyano, $C_{1-4}$ alkyl, $C_{1-4}$ deuterated alkyl, or $C_{1-4}$ fluoroalkyl;
n1 is an integer selected from 0 to 6.

**12.** The compound according to any one of claims 1-11, **characterized in that** the compound is selected from:

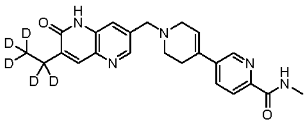 , 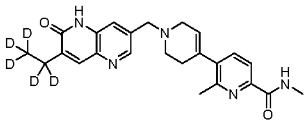 , 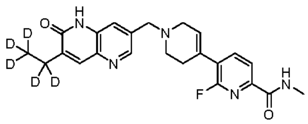 ,

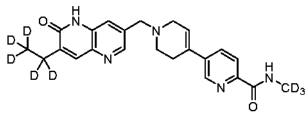 , 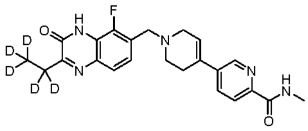 or 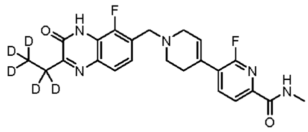 .

**13.** The compound according to any one of claims 1 to 11, **characterized in that** the compound is selected from:

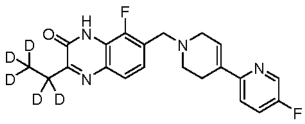 or 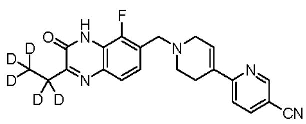 .

**14.** The compound according to any one of claims 1 to 11, **characterized in that** the compound is selected from:

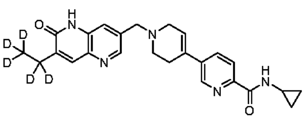 , 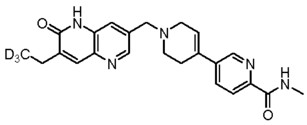 , 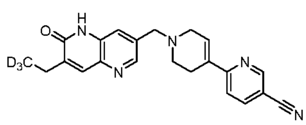

or

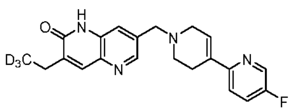 .

**15.** A pharmaceutical composition, **characterized in that** it comprises: the compound according to any one of claims 1 to 14, or a pharmaceutically acceptable salt, ester, stereoisomer, tautomer, polymorph, solvate, N-oxide, isotopically labeled compound, metabolite, or prodrug thereof, as an active ingredient; and a pharmaceutically acceptable carrier.

**16.** The compound according to any one of claims 1 to 14, or a pharmaceutically acceptable salt, ester, stereoisomer, tautomer, polymorph, solvate, N-oxide, isotopically labeled compound, metabolite, or prodrug thereof, and the pharmaceutical composition according to claim 15, intended for use in the manufacture of a medicament for preventing and/or treating a PARP1 enzyme-related disease.

**17.** The use according to claim 16, **characterized in that** the PARP1 enzyme-related disease is a neoplastic disorder.

**18.** The use according to claim 17, **characterized in that** the neoplastic disorder lacks a HR-dependent repair pathway for DNA DSB.

**19.** The use according to claim 17 or 18, **characterized in that** the neoplastic disorder comprises one or more types of cancer cells having a reduced or absent ability to repair DNA DSBs by HR compared to normal cells.

**20.** The use according to claim 19, **characterized in that** the cancer cells have a BRCA1 or BRCA2 deficient phenotype.

**21.** The use according to any one of claims 17 to 20, **characterized in that** the neoplastic disorder includes breast cancer, ovarian cancer, primary peritoneal cancer, pancreatic cancer, prostate cancer, hematological cancer, gastrointestinal cancer, glioblastoma, or lung cancer.

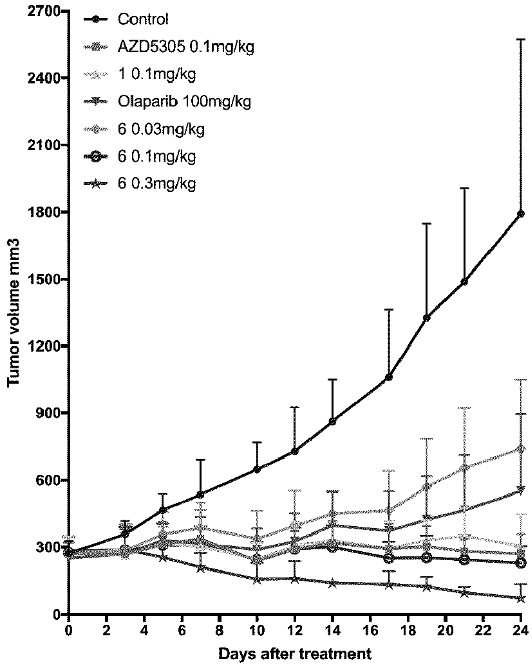
Control
AZD5305 0.1mg/kg
1 0.1mg/kg
Olaparib 100mg/kg
6 0.03mg/kg
6 0.1mg/kg
6 0.3mg/kg
Tumor volume mm3
Days after treatment

Fig. 1

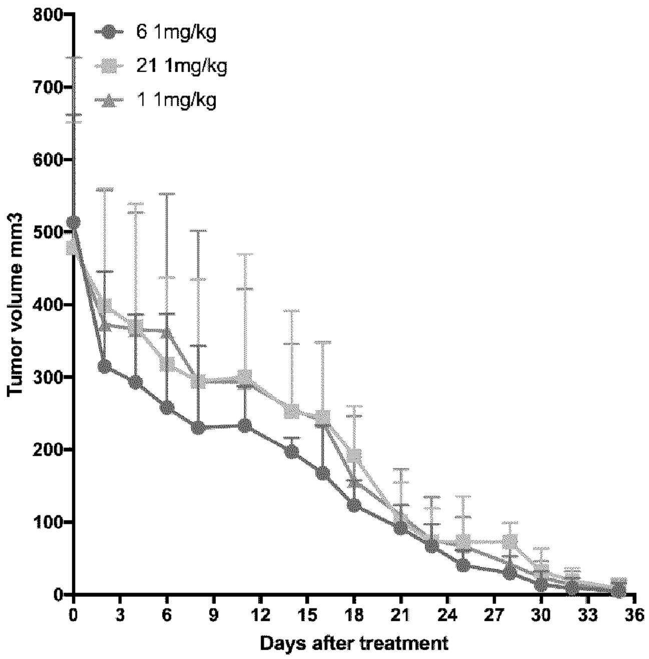
6 1mg/kg
21 1mg/kg
1 1mg/kg
Tumor volume mm3
Days after treatment

Fig. 2

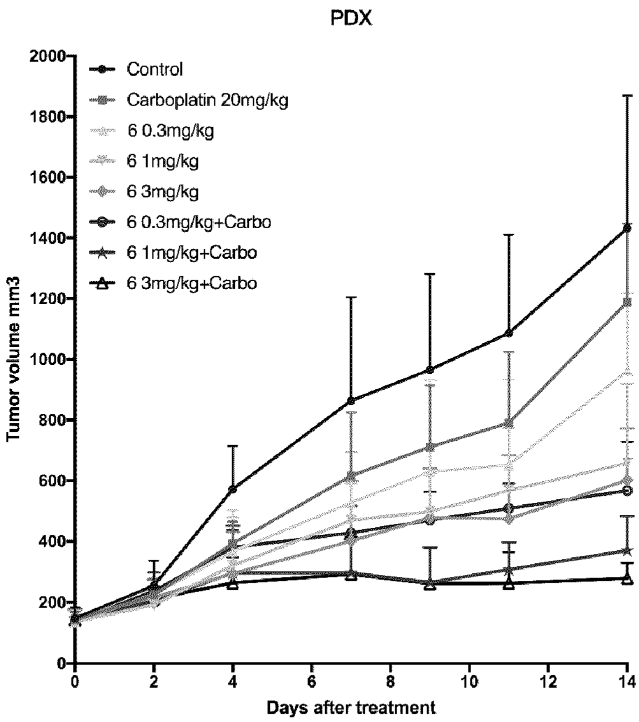
PDX
Control
Carboplatin 20mg/kg
6 0.3mg/kg
6 1mg/kg
6 3mg/kg
6 0.3mg/kg+Carbo
6 1mg/kg+Carbo
6 3mg/kg+Carbo
Tumor volume mm3
Days after treatment
0
200
400
600
800
1000
1200
1400
1600
1800
2000
0
2
4
6
8
10
12
14

Fig. 3

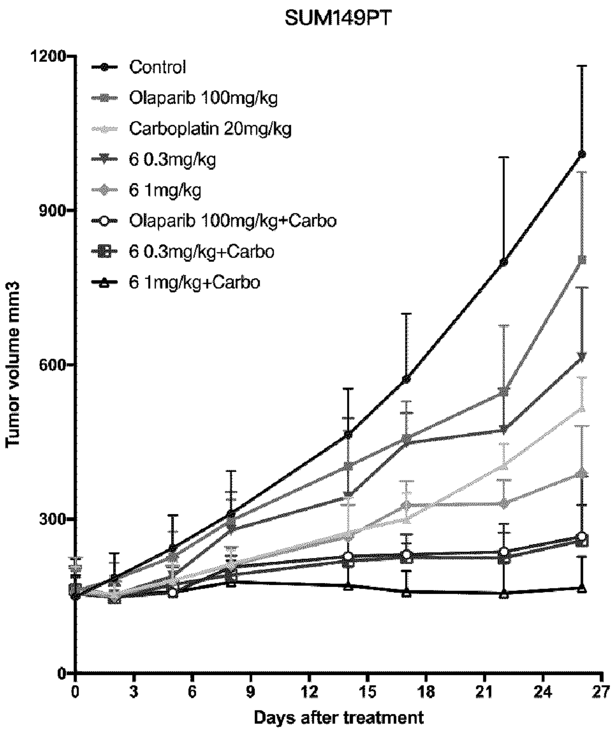
SUM149PT
Control
Olaparib 100mg/kg
Carboplatin 20mg/kg
6 0.3mg/kg
6 1mg/kg
Olaparib 100mg/kg+Carbo
6 0.3mg/kg+Carbo
6 1mg/kg+Carbo
Tumor volume mm3
Days after treatment
0
300
600
900
1200
0
3
6
9
12
15
18
21
24
27

Fig. 4

**INTERNATIONAL SEARCH REPORT**

International application No.
**PCT/CN2023/125496**

**A. CLASSIFICATION OF SUBJECT MATTER**

C07D471/04(2006.01)i；C07D403/14(2006.01)i；C07D401/14(2006.01)i；A61K31/444(2006.01)i；A61P35/00(2006.01)i；A61P35/02(2006.01)i；A61P35/04(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

CNABS, CNTXT, EPTXT, ISI web of knowledge, STN: 赜灵生物医药, 抑制剂, 吡啶, ZENITAR BIOMEDICAL, INHIBITORS, PYRIDINE, PARP, 结构式检索, structural formula search.

**C. DOCUMENTS CONSIDERED TO BE RELEVANT**

| Category* | Citation of document, with indication, where appropriate, of the relevant passages | Relevant to claim No. |
|---|---|---|
| PX | CN 115232154 A (SHANGHAI HANSOH BIOMEDICAL CO., LTD.) 25 October 2022 (2022-10-25)<br>claims 1-14, and embodiments | 1-21 |
| PX | WO 2022228387 A1 (FOCHON BIOSCIENCES, LTD.) 03 November 2022 (2022-11-03)<br>claims 1-23, and embodiments | 1-21 |
| PX | WO 2023046034 A1 (MINGHUI PHARMACEUTICAL (HANGZHOU) LTD. et al.) 30 March 2023 (2023-03-30)<br>claims 1-16, and embodiments | 1-21 |
| PX | WO 2023051812 A1 (HAISCO PHARMACEUTICAL GROUP CO., LTD.) 06 April 2023 (2023-04-06)<br>claims 1-16, and embodiments | 1-21 |
| PX | WO 2023109521 A1 (KEYTHERA (SUZHOU) BIO-PHARMACEUTICALS CO., LTD.) 22 June 2023 (2023-06-22)<br>claims 1-14, and embodiments | 1-21 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 January 2024** | **26 January 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2023/125496**

**C. DOCUMENTS CONSIDERED TO BE RELEVANT**

| Category* | Citation of document, with indication, where appropriate, of the relevant passages | Relevant to claim No. |
|---|---|---|
| PX | CN 116891456 A (SHANGHAI HANSOH BIOMEDICAL CO., LTD. et al.) 17 October 2023 (2023-10-17)<br>claims 1-13, and embodiments | 1-21 |
| A | CN 114144413 A (ASTRAZENECA AB) 04 March 2022 (2022-03-04)<br>claims 1-37, and embodiments | 1-21 |
| A | CN 102341394 A (TAKEDA PHARMACEUTICAL CO., LTD.) 01 February 2012 (2012-02-01)<br>claims 1-40, and embodiments | 1-21 |
| A | WO 2009053373 A1 (JANSSEN PHARMACEUTICA N.V. et al.) 30 April 2009 (2009-04-30)<br>claims 1-13, and embodiments | 1-21 |

Form PCT/ISA/210 (second sheet) (July 2022)

**INTERNATIONAL SEARCH REPORT**
**Information on patent family members**

International application No.
**PCT/CN2023/125496**

| Patent document cited in search report | Publication date (day/month/year) | Patent family member(s) | Publication date (day/month/year) |
|---|---|---|---|
| CN 115232154 A | 25 October 2022 | TW 202309026 A | 01 March 2023 |
| | | CA 3215823 A1 | 27 October 2022 |
| | | WO 2022223025 A1 | 27 October 2022 |
| | | BR 112023019797 A2 | 07 November 2023 |
| | | AU 2022261029 A1 | 19 October 2023 |
| | | IL 307824 A | 01 December 2023 |
| WO 2022228387 A1 | 03 November 2022 | TW 202309027 A | 01 March 2023 |
| WO 2023046034 A1 | 30 March 2023 | None | |
| WO 2023051812 A1 | 06 April 2023 | None | |
| WO 2023109521 A1 | 22 June 2023 | TW 202327591 A | 16 July 2023 |
| CN 116891456 A | 17 October 2023 | None | |
| CN 114144413 A | 04 March 2022 | AU 2020318599 A1 | 10 March 2022 |
| | | AU 2020318599 B2 | 07 September 2023 |
| | | US 2021040084 A1 | 11 February 2021 |
| | | US 11325906 B2 | 10 May 2022 |
| | | UY 38793 A | 26 February 2021 |
| | | CR 20220070 A | 21 March 2022 |
| | | ECSP 22012826 A | 31 March 2022 |
| | | BR 112022000534 A2 | 10 May 2022 |
| | | MX 2022000711 A | 23 February 2022 |
| | | AR 119424 A1 | 15 December 2021 |
| | | IL 289534 A | 01 March 2022 |
| | | KR 20220035941 A | 22 March 2022 |
| | | CL 2022000110 A1 | 20 September 2022 |
| | | JP 2022541483 A | 26 September 2022 |
| | | JOP 20220008 A1 | 30 January 2023 |
| | | DOP 2022000006 A | 15 March 2022 |
| | | PE 20221339 A1 | 13 September 2022 |
| | | CO 2022001590 A2 | 18 March 2022 |
| | | US 2022227768 A1 | 21 July 2022 |
| | | TW 202116750 A | 01 May 2021 |
| | | CA 3145644 A1 | 28 January 2021 |
| | | WO 2021013735 A1 | 28 January 2021 |
| | | EP 3999506 A1 | 25 May 2022 |
| CN 102341394 A | 01 February 2012 | ECSP 11011284 A | 31 October 2011 |
| | | CR 20110452 A | 28 February 2012 |
| | | WO 2010085570 A1 | 29 July 2010 |
| | | CL 2011001754 A1 | 20 January 2012 |
| | | US 2013274239 A1 | 17 October 2013 |
| | | US 8822470 B2 | 02 September 2014 |
| | | JP 2012515786 A | 12 July 2012 |
| | | JP 5567599 B2 | 06 August 2014 |
| | | AU 2010206744 A1 | 04 August 2011 |
| | | AU 2010206744 B2 | 20 August 2015 |
| | | SG 172958 A1 | 29 August 2011 |
| | | CA 2750106 A1 | 29 July 2010 |
| | | DOP 2011000237 A | 15 September 2011 |
| | | EP 2389379 A1 | 30 November 2011 |
| | | CO 6410305 A2 | 30 March 2012 |

Form PCT/ISA/210 (patent family annex) (July 2022)

**INTERNATIONAL SEARCH REPORT**
**Information on patent family members**

International application No.
**PCT/CN2023/125496**

| Patent document cited in search report | Publication date (day/month/year) | Patent family member(s) | Publication date (day/month/year) |
|---|---|---|---|
| | | US 2015031652 A1 | 29 January 2015 |
| | | US 9187497 B2 | 17 November 2015 |
| | | US 2011158989 A1 | 30 June 2011 |
| | | US 8124606 B2 | 28 February 2012 |
| | | EA 201170963 A1 | 30 March 2012 |
| | | EA 020301 B1 | 30 October 2014 |
| | | KR 20110107396 A | 30 September 2011 |
| | | KR 101779137 B1 | 18 September 2017 |
| | | MY 152386 A | 15 September 2014 |
| | | BRPI 1007358 A2 | 06 March 2018 |
| | | IL 213993 A0 | 31 August 2011 |
| | | IL 213993 A | 30 March 2017 |
| | | PE 20120418 A1 | 04 May 2012 |
| | | MX 2011007741 A | 06 September 2011 |
| | | NZ 594322 A | 25 January 2013 |
| | | TN 2011000339 A1 | 27 March 2013 |
| | | US 2010190763 A1 | 29 July 2010 |
| | | US 7928105 B2 | 19 April 2011 |
| | | MA 33053 B1 | 01 February 2012 |
| | | US 2012122835 A1 | 17 May 2012 |
| | | US 8450323 B2 | 28 May 2013 |
| WO 2009053373 A1 | 30 April 2009 | PL 2215075 T3 | 30 April 2014 |
| | | US 2010222348 A1 | 02 September 2010 |
| | | US 8404713 B2 | 26 March 2013 |
| | | ES 2448870 T3 | 17 March 2014 |
| | | EP 2215075 A1 | 11 August 2010 |
| | | EP 2215075 B1 | 11 December 2013 |
| | | JP 2011500758 A | 06 January 2011 |
| | | JP 5525447 B2 | 18 June 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **FARMER H** ; **MCCABE N et al.** Targeting the DNA repair defect in BRCA mutant cells as a therapeutic strategy[J. *Nature*, 2005, vol. 434 (7035), 917-921
- **BRYANT, H.** ; **SCHULTZ, N.** ; **THOMAS, H. et al.** Specific killing of BRCA2-deficient tumors with inhibitors of poly(ADP-ribose) polymerase. *Nature*, 2005, vol. 434, 913-917
- **HARRIS P A** ; **BOLOOR A** ; **CHEUNG M et al.** Discovery of 5-[[4-[(2,3-dimethyl-2H-indazol-6-yl) methylamino]-2-pyrimidinyl]amino]-2-methyl-benzenesulfonamide (Pazopanib), a novel and potent vascular endothelial growth factor receptor inhibitor. [J].. *Journal of Medicinal Chemistry*, 2008, vol. 51 (15), 4632
- Remington's Pharmaceutical Sciences. Mack Publishing Company, 2005
- Handbook of Pharmaceutical Salts: Properties, Selection, and Use. Wiley-VCH, 2002
- **T.L. GILCHRIST**. Comprehensive Organic Synthesis. Academic Press, vol. 7, 748-750
- **G.W.H. CHEESEMAN** ; **E.S.G WERSTIUK**. Advances in Heterocyclic Chemistry. Academic Press, vol. 22, 390-392
- **T. HIGUCHI** ; **V. STELLA**. Pro-drugs as Novel Delivery Systems,. *ACS Symposium Series*, vol. 14
- **H. BUNDGAARD**. Design of Prodrugs. Elsevier, 1985
- *Technical Guidelines for Non-clinical Pharmacokinetic Studies of Chemical Drugs*, 2014